# EUROPEAN PATENT APPLICATION

(11) **EP 3 403 652 A1**
(43) Date of publication of application: **21.11.2018**
(21) Application number: 17171737.4
(22) Date of filing: 18.05.2017
(51) Int. Cl.: A61K 31/429, A61K 31/435, A61K 31/4545, A61K 31/609, A61P 13/12

(54) **PREVENTION AND TREATMENT OF FIBROBLAST GROWTH FACTOR 23 (FGF23)-ASSOCIATED DISORDERS INCLUDING CHRONIC KIDNEY DISEASE (CKD)**

(71) Applicant: Veterinärmedizinische Universität Wien, 1210 Vienna (AT)
(72) Inventor: ERBEN, Reinhold G., 2531 Gaaden (AT); ANDRUKHOVA, Olena, 2232 Deutsch-Wagram (AT)
(74) Representative: Sonn & Partner Patentanwälte

(57) **Abstract**

The invention discloses WNK signalling inhibitors, in particular closantel, for use in the prevention and treatment of disorders caused and/or characterized by elevated levels of serum fibroblast growth factor (FGF23), especially chronic kidney disease (CKD). The invention also provides pharmaceutical compositions comprising one/or more WNK signalling inhibitors and methods for use said pharmaceutical compositions in the treatment of subjects diagnosed with FGF23-associated disorders, wherein subjects may be humans and/or companion animals, e.g. dogs and cats.

## Description

The present invention relates to compositions and methods for the prevention and treatment of disorders characterized and/or caused by high levels of fibroblast growth factor 23 (FGF23), especially chronic kidney disease (CKD).

Chronic kidney disease (CKD), or chronic renal disease, is a chronic condition characterized by progressive loss of kidney function over a period of months to years and describes all stages (1-5) of decreased renal function ranging from mild and moderate to severe chronic kidney failure (end-stage kidney disease requiring dialysis). CKD is classified according to the Kidney Disease Improving Global Outcomes (KDIGO) based on the glomerular filtration rate (GFR) and proteinuria (condition characterized by an excess amount of protein in the urine). GFR describes the flow rate of filtered fluid through the kidney and is calculated based on age, sex and blood creatinine.

CKD is an important public health problem associated with a number of serious complications, including increased incidence of cardiovascular disease, anemia and metabolic bone disease. The most common causes for developing CKD are diabetes, high blood pressure and genetic predisposition, which are responsible for about two-thirds of the cases.

Early stages of CKD often remain undiagnosed due to patients being asymptomatic. Early detection and treatment of CKD however is vital to prevent progression of renal function decline and to reduce sequelae such as cardiovascular and metabolic disease. Over the last decade, substantial evidence has been accumulated regarding the role of high circulating levels of fibroblast growth factor 23 (FGF23) as a predictor of adverse outcomes in patients with CKD at all stages.

FGF23 is a phosphaturic hormone secreted from bone cells, which together with PTH and the biologically active vitamin D hormone 1α,25-dihydroxyvitamin D3 [1,25(OH)2D3], is a major systemic regulator of phosphate homeostasis. In the kidney, FGF23 reduces phosphate reabsorption from urine by a downregulation of sodium phosphate co-transporters (2a and 2c) in proximal renal tubular epithelium. In addition, FGF23 downregulates renal 1α-hydroxylase expression, thereby suppressing the production of 1α,25-dihydroxyvitamin D3.

FGF23 was first described in the context of several hereditary and acquired human diseases (Ruppe MD. X-Linked Hypophosphatemia. 2012 Feb 9 [Updated 2014 Oct 16]. In: Pagon RA, Adam MP, Ardinger HH, et al., editors. GeneReviews® [Internet]. Seattle (WA): University of Washington, Seattle; 1993-2017). Autosomal dominant hypophosphatemic rickets (ADHR) is a rare genetic renal phosphate-wasting disorder caused by a missense mutation in the FGF23 gene, which results in the stabilization of the active form of the protein leading to prolonged or enhanced FGF23 activity. In contrast, tumor-induced osteomalacia (TIO), also known as oncogenic osteomalacia (OOM), is an acquired paraneoplastic syndrome caused by high levels of serum FGF23 that are secreted by mesenchymal tumors. Further disorders associated with high levels of FGF23 and phosphate-wasting are the McCune-Albright syndrome and linear nevus sebaceous syndrome (or epidermal nevus syndrome). The hypophosphatemic rickets observed in McCune-Albright syndrome are associated with overproduction of FGF23 by the fibrous dysplastic bone resulting in renal phosphate wasting. Hypophosphatemia in linear nevus sebaceous syndrome, which is characterized by multiple cutaneous nevi, is due to excessive production of FGF23 by fibrous dysplasia. Excessive FGF23 levels are also associated with acute diseases such as acute myocardial infarction and acute kidney injury (Schnedl C et al., Disease Markers, 2015. Article ID 358086).

Generally, increased serum levels of FGF23 result in hypophosphatemia, rickets and/or ostemalacia, mainly because defects in phosphate metabolism lead to poor bone mineralization and fractures. In a study performed in CKD patients, Pande S et al. (Nephron Physiol., 2006. 104(1): p23-p32) demonstrated that FGF23 levels rapidly decreased after kidney transplantation suggesting that FGF23 is cleared by the kidney. In contrast, loss of FGF23 in mice results in hyperphosphatemia and hypervitaminosis D further highlighting the important function of FGF23 in regulating the phosphate metabolism.

In CKD patients, serum FGF23 concentrations rise in parallel with the decline in glomerular filtration rate (GFR), and can reach levels 1,000-fold above normal in more advanced renal failure. The reason why FGF23 increases during CKD progression is not entirely clear. It has been shown that the increase in circulating intact FGF23 occurs independently of hyperphosphatemia and increased parathyroid hormone (PTH) in early CKD stages. Therefore, other factors such as reduced renal elimination of FGF23, or increased secretion of FGF23-stimulating substances such as aldosterone or proinflammatory cytokines may be involved in the upregulation of blood concentrations of intact FGF23 in CKD. Prospective studies in CKD patients have demonstrated that elevated FGF23 levels are independently associated with CKD progression, left ventricular hypertrophy (LVH), cardiovascular risk, and all-cause mortality.

In typical FGF23 target tissues such as the kidney, the biological effects of FGF23 are mediated through a receptor complex consisting of FGF receptors (FGFRs) and of the co-receptor αKlotho (Klotho). FGF23 is not only a phosphaturic, but also a calcium (Ca²⁺) - and sodium (Na⁺) -conserving hormone. FGF23 directly increases the apical membrane abundance of the epithelial Ca²⁺ channel transient receptor potential vanilloid-5 (TRPV5) and of the Na⁺:Cl co-transporter (NCC) in distal renal tubules through a signalling cascade involving FGFR1c/Klotho receptor complex, extracellular signal-regulated kinase 1 and 2 (ERK1/2), serum/glucocorticoid-regulated kinase-1 (SGK1), and with-no lysine kinase-4 (WNK4).

WNKs are key regulators of intracellular transport of membrane proteins, thereby acting as a complex of WNK1, 3, and 4. WNKs bind to and stimulate the kinases SPAK (STE20/SPS1-related proline/alanine-rich kinase) and OSR1 (oxidative stress-responsive kinase 1), which directly phosphorylate and stimulate Cl⁻-importing, Na⁺-driven cation-chloride cotransporters(CCCs) or inhibit the Cl⁻-extruding, K⁺-driven CCCs. The inventors have previously shown that FGF23 is involved in the physiological regulation of Ca²⁺ and Na⁺ reabsorption in renal distal tubules by Klotho-dependent activation of WNK4 (Andrukhova O et al., EM-BO J., 2014. 33, 229-246; Andrukhova O et al., EMBO Mol. Med., 2014. 6, 744-759).

WNKs have a vital role in the control of the blood pressure through regulation of the NCC and NKCC1/2 co-transporters (Richardson C & Alessi D R, J. Cell Sci., 2008. 121, 3293-3304). Therefore, the link between FGF23 and volume homeostasis and blood pressure regulation may at least partially explain the association between circulating FGF23 and cardiovascular risk in CKD.

Interestingly, the inventors of the present invention found that the FGF23-induced WNK activation with subsequent Na⁺ retention, volume overload and hypertension is the key driving factor underlying the pro-hypertrophic effects of excessive circulating Fgf23 in CKD (Andrukhova O et al., EMBO Mol. Med., 2014. 6, 744-759). The finding was supported by epidemiological studies that have linked elevated circulating FGF23 levels with hypercalcemia and hypernatremia in CKD progression (Pavik I et al., Nephrol. Dial. Transplant., 2013. 28, 352-359; Russo D & Battaglia Y, Int. J. Nephrol., 2011. 364890). Several clinical studies further confirmed the association between circulating FGF23, volume overload and adverse cardiac outcomes in CKD patients (Hung S C et al., J. Am. Heart Assoc., 2015. 4; Unver S et al., Ren Fail. 37, 2015. 951-956).

Furthermore, by employing genetic loss-of-function models in Fgf23 and Klotho deficient mice together with pharmacological FGF23 inhibition models, the inventors of the present invention have recently shown that FGF23 signalling plays a pivotal role in the pathogenesis of CKD, whereby activation of WNK signalling turned out to be the disease-driving factor.

They have shown that low dose anti-FGF23 Ab treatment substantially ameliorated disease progression and left ventricular dysfunction by partially preventing volume overload, hypertension, and vascular calcification in CKD mice. In contrast, Shalhoub V et al. (J. Clin. Invest, 2012. 122, 2543-2553) previously reported that high dose anti-FGF23Ab therapy increased mortality and vascular calcifications in CKD rats on a high phosphate diet. The discrepant findings can probably be explained by the fact that complete elimination of Fgf23 function by high dose anti-FGF23 Ab leads to a Fgf23 deficiency-like phenotype characterized by inappropriately high production of 1,25(OH)2D3, and subsequent hypercalcemia and hyperphosphatemia.

In order to exploit the strikingly beneficial effects of lowering blood Fgf23 concentrations in CKD, it seems necessary to carefully titrate the dose of anti-FGF23 Ab to prevent the toxic effects of over-suppression. Because the toxic effect of anti-FGF23 Ab therapy is mainly based on reduction of the suppressive effect of FGF23 on phosphate reabsorption and 1,25(OH)2D3 production in proximal renal tubules, the inventors found a safer therapeutic strategy with a wider therapeutic window by selectively interfering with the detrimental effects of excessive FGF23 signalling in distal renal tubules by inhibiting WNK signalling.

Therefore, to overcome the adverse effects of FGF23 targeting therapies, new therapeutic strategies for patients diagnosed with disorders associated with elevated serum FGF23 levels, especially CKD patients, are needed.

It is an object of the present invention to provide means and methods for use in preventing and treating disorders caused by elevated levels of serum FGF23, especially chronic kidney disease (CKD).

The present invention therefore relates to an agent used to lower blood FGF23 concentrations in subjects with a disorder associated with or caused by elevated serum FGF23 levels, in particular subjects with CKD. According to the present invention, it was surprisingly found that WNK signalling inhibitors, in particular closantel, are potent agents to reduce the adverse FGF23-mediated WNK signaling activation in the kidney in vivo. The inventors have further shown for the first time that administration of a WNK signalling inhibitor prevented CKD progression in mice. Therefore, it is an important feature of the present invention that its administration is not only suited for late stage CKD, but also for early stages of CKD. It therefore is an object of the present invention to also provide prevention of disease progression in a subject diagnosed with a disorder that is caused and/or characterized by excessive levels of serum FGF23.

Several possible strategies for inhibiting the WNK-SPAK/OSRI signalling cascade have been proposed (Alessi D R et al., Sci Signal., 2014. 7, 334). One possible approach is to directly inhibit the kinase activity of either the WNK or the SPAK/OSRI kinases. For example, Yamada, K et al. (Nature Chem Biology, 2016., 12(11), 896-898) discloses an orally bioavailable inhibitor of WNK1 and WNK4, called WNK463, that decreases SPAK and OSR1 phosphorylation in a dose-dependent manner. Indirect inhibition through targeting WNK upstream regulators, e.g. KLHL3, or SPAK/OSR1 scaffold proteins, MO25 α or β isoforms, were also proposed, however the underlying mechanism and specificity is not yet fully understood. Thus far, inhibition of the interaction between WNKs and SPAK/OSRI by targeting the CCT domain within SPAK/OSRI seems to be the most selective approach of WNK pathway inhibition. Mori T et al. (Biochem J, 2013. 455, 339-345) performed high-throughput screening of 17000 compounds and discovered two novel WNK signalling inhibitors (STOCK1S-50699 and STOCK2S-26016) preventing SPAK phosphorylation in a dose-dependent manner by preventing WNK-SPAK binding.

In a compound library screening approach, Kikuchi E et al. (J Am Soc Nephrol, 2014. 1046-6673/2608) further identified STOCK1S-14279 (N-[4-(1-bromonaphthalen-2-yl)oxy-3-chlorophenyl]-3,5-dichloro-2-hydroxybenzamide) and closantel (N-[5-chloro-4-[(4-chlorophenyl)-cyanomethyl]-2-methylphenyl]-2-hydroxy-3,5-diiodobenzamide) as potent inhibitors of the WNK-regulated kinase SPAK (Ste20-like proline/alanine-rich kinase).

Targeting the WNK signalling pathway in a therapeutic manner was described before. For example, WO 03/007793 A2 discloses mammalian WNK inhibitors, e.g. anti-WNK antibodies or isolated nucleic acids complementary to isolated nucleic acids encoding a human WNK, for use in a method of treating human patients with hypertension and pseudohypoaldosteronism type II. A similar approach of targeting WNK is described in JP 2007/074951 A, wherein the interaction of WNK1 and WNK4 with SPAK is disclosed and the inhibition of their interactions for treating hypertension proposed. Furthermore, US 2009/0203012 A1 discloses methods comprising identifying genetic predisposition for developing a metabolic syndrome, screening compounds that inhibit the kinase activity of STK39/SPAK and using said inhibitory compounds for treating a metabolic syndrome, hypertension, cardiovascular disease and T2D. According to the prior art, WNK signalling inhibition is obviously beneficial in the treatment of metabolic diseases. However, nothing in the prior art so far provided a link between WNK signalling inhibition as therapy for disorders caused and/or characterized by excessive FGF23 levels, especially CKD.

According to preferred embodiments of the present invention, the WNK signalling inhibitor used to reduce the adverse effects of increased renal FGF23 signalling in subjects with a disorder associated with or caused by elevated serum FGF23 levels, is closantel (N-[5-chloro-4-[(4-chlorophenyl)-cyanomethyl]-2-methylphenyl]-2-hydroxy-3,5-diiodobenzamide), STOCK1S-14279 (N-[4-(1-bromonaphthalen-2-yl)oxy-3-chlorophenyl]-3,5-dichloro-2-hydroxybenzamide), STOCK1S-50699 ((2Z)-3-ethyl-2-[[(3Z)-3-[(E)-3-(3-ethyl-1,3-benzothiazol-3-ium-2-yl)prop-2-enylidene]-5-methylcyclohexen-1-yl]methylidene]-1,3-benzothiazole), STOCK2S-26016 (7-Ethoxy-N3-(2-furanylmethyl)-3,9-acridinediamine), WNK463 (N-tert-butyl-3-[1-[5-[5-(trifluoromethyl)-1,3,4-oxadiazol-2-yl]pyridin-2-yl]piperidin-4-yl]imidazole-4-carboxamide), or any combination thereof.

Closantel (N-[5-chloro-4-[(4-chlorophenyl)-cyanomethyl]-2-methylphenyl]-2-hydroxy-3,5-diiodobenzamide) is known as an anthelmintic from the group of salicylanilide. Salicylanilides are important anti-parasitics in the control of Haemonchus spp. and Fasciola spp. infestation in sheep and cattle, and Oestrus ovis in sheep. Macielag M J et al. (J. Med. Chem., 1998. 41 (16), 2939-45) tested for antibacterial activity of closantel and related derivatives against the drug-resistant organisms, methicillin-resistant Staphylococcus aureus (MRSA) and vancomycinresistant Enterococcus faecium (VREF). Furthermore, WO 2016/193136 A1 discloses halogenated salicylanilides, e.g. closantel, or pharmaceutically acceptable salts or esters thereof, for use in treating infections caused by Clostridium bacteria, especially diarrhoea and colitis in humans and warm-blooded animals. Its suitability for topical treatment or prevention of Staphylococcus and Streptococcus infections is disclosed in WO 2016/038035 A1.

The present invention therefore provides a new unexpected therapeutic area for WNK signalling inhibitors, especially closantel. In particular, the present invention discloses WNK signalling inhibitors and/or pharmaceutic compositions thereof for the use in preventing and/or treating disorder caused and/or characterized by elevated serum FGF23 levels, especially CKD.

In the context of the present invention, disorders caused and/or characterized by elevated serum FGF23 levels are preferably selected from the group consisting of tumor-induced osteomalacia (TIO), X-linked hypophosphatemia, autosomal dominant hypophosphatemic rickets (ADHR), McCune-Albright syndrome, linear nevus sebaceous syndrome, acute myocardial infarction, and CKD, especially CKD.

According to a preferred embodiment, the present invention is used for preventing and/or treating subjects with CKD. Importantly, the present invention provides treatment for CKD subjects in any stage of the disease process, wherein CKD stages are classified according to the KDIGO GFR system (KDIGO 2012 Clinical Practice Guideline for Evaluation and Management of Chronic Kidney Disease, Volume 3, Issue 1, January 2013).

According to the present invention, the subject having a disorder caused and/or characterized by elevated levels of serum FGF23 may be human and/or a non-human animal, whereby the non-human animal is preferably a domestic companion animal, e.g. a dog or a cat.

According to the present invention, the WNK signalling inhibitor used for prevention and/or treatment of disorders caused and/or characterized by elevated serum FGF23 levels, may be formulated in a pharmaceutical composition comprising at least one, preferably more than one bioactive WNK signalling inhibitor selected from the group consisting of closantel (N-[5-chloro-4-[(4-chlorophenyl)-cyanomethyl]-2-methylphenyl]-2-hydroxy-3,5-diiodobenzamide), STOCK1S-14279 (N-[4-(1-bromonaphthalen-2-yl)oxy-3-chlorophenyl]-3,5-dichloro-2-hydroxybenzamide), STOCK1S-50699 ((2Z)-3-ethyl-2-[[(3Z)-3-[(E)-3-(3-ethyl-1,3-benzothiazol-3-ium-2-yl)prop-2-enylidene]-5-methylcyclohexen-1-yl]methylidene]-1,3-benzothiazole), STOCK2S-26016 (7-Ethoxy-N3-(2-furanylmethyl)-3,9-acridinediamine) or WNK463 (N-tert-butyl-3-[1-[5-[5-(trifluoromethyl)-1,3,4-oxadiazol-2-yl]pyridin-2-yl]piperidin-4-yl]imidazole-4-carboxamide), or pharmaceutically acceptable salts thereof, preferably closantel, more preferred a pharmaceutically acceptable salt of closantel.

The WNK signalling inhibitor, a pharmaceutically acceptable salt thereof, or the composition comprising a WNK signalling inhibitor or its salt, wherein the WNK signalling inhibitor is preferably closantel or a pharmaceutically acceptable salt thereof, may be administered in an amount, frequency, and duration that measurably decreases levels of serum FGF23. Preferably, closantel or a pharmaceutically acceptable salt thereof is administered in an amount between 0.01 and 3000 mg/day, more preferably, in an amount between 0.1 and 2000 mg/day (WO 2004/006906 A).

In some embodiments of the present invention, the composition further comprises a pharmaceutically acceptable excipient and/or solvent. The pharmaceutically acceptable excipient may be selected from the group consisting of, but not limited to, surfactants, preferably oleic acid, thickening agents, salts, buffers, or any other inert excipient commonly used in pharmaceutical industry.

Pharmaceutically acceptable solvents according to the inventive composition comprising WNK signalling inhibitors are alcohols from the group consisting of ethanol, benzyl alcohol, isopropanol, butanol, or a mixture thereof, with ethanol, benzyl alcohol or a mixture thereof being preferred. In another embodiment, the pharmaceutical composition comprising the WNK signalling inhibitor may be dissolved in propylene glycol, diethylene glycol monoethyl ether (transcutol), ethylene glycol, butylene glycol, polyvinylpyrrolidone (PVP), polyoxypropylene/polyoxyethylene, polyethylene glycol, or the like, preferably propylene glycol, diethylene glycol monoethyl ether or a mixture thereof.

In another embodiment of the present invention the pharmaceutical composition comprising one or more WNK inhibitors, or pharmaceutically acceptable salts thereof, and one or more pharmaceutically acceptable excipient and/or solvent, is administered orally or parenterally for preventing and/or treating conditions caused/or characterized by elevated serum FGF23 levels, especially CKD.

The pharmaceutical composition according to the present invention may be in form of tablets, lozenges, hard or soft capsules, aqueous or oily suspensions, emulsions, dispersible powders or granules, syrups or elixirs suitable for oral administration. In another embodiment, the inventive composition is in form of a sterile aqueous or oily solution suitable for parenteral administration, e.g. intravenous, subcutaneous, intramuscular or intraperitoneal injection.

The present invention also features a method for treating a subject having a disorder caused and/or characterized by elevated serum FGF23 levels, especially CKD. This method includes the step of administering to the subject a WNK signalling inhibitor, or a salt thereof, in an amount effective to decrease FGF23-induced WNK signalling in the kidney. Accordingly, the WNK signalling inhibitor may be administered as single agent or pharmaceutical composition, preferably as pharmaceutical composition. Desirable routes of administration of the single agent or pharmaceutical composition according to the present invention include enteral and parenteral administration, preferably oral or intravenous administration. The subject treated by the inventive method may be a human and/or a non-human animal, in particular a domesticated companion animal. Non-limiting examples of domesticated companion animals are dogs and cats.

In another embodiment, the inventive pharmaceutical composition is for use in a method for treating a subject that suffers from a disorder associated with high levels of serum FGF23. Examples are subjects diagnosed with tumor-induced osteomalacia (TIO), X-linked hypophosphatemia, autosomal dominant hypophosphatemic rickets (ADHR), McCune-Albright syndrome, linear nevus sebaceous syndrome, and acute myocardial infarction.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

As used herein, the term "WNK signalling inhibitor" refers to a compound preventing phosphorylation of SPAK and/or OSR1 kinases, either directly or indirectly. For example, WO 2007/010210 A2 discloses a compound-screening assay for identifying WNK signalling modulators that prevent phosphorylation of WNK substrates, SPAK or OSRI.

The term "disorder" refers to a medical health condition associated with adverse effects on the well-being of a subject and resulting in illness.

As used herein, the term "subject" refers to any living organism unless otherwise specified. In specific examples, subjects include: humans and non-human animals; domestic mammals such as dogs and cats. The term does not denote a particular age or sex. Thus, adult and newborn subjects, as well as fetuses, whether male or female, are intended to be covered.

As used herein, the term "enteral administration" refers to any route of administration involving the esophagus, stomach, and small and large intestines (i.e., the gastrointestinal tract). Methods of enteral administration thus include oral, sublingual (dissolving the drug under the tongue), and rectal.

The term "parenteral administration" refers to any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intraperitoneal, intramuscular, intrasternal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multi-dose containers containing a preservative. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. For parenteral administration, the active ingredient may also be provided in dry (i.e. powder or granular) form for reconstitution with a suitable vehicle (e. g. sterile pyrogen-free water) prior to parenteral administration of the reconstituted composition.

The term "pharmaceutical composition" refers to a formulation comprising a biologically active ingredient and suitable pharmaceutically acceptable inert substances, such as carriers, excipients and/or solvents.

The term "pharmaceutically acceptable", such as pharmaceutically acceptable carrier, excipient, etc. refers to pharmacologically acceptable and substantially non-toxic to the subject to which the particular compound is administered.

The term "pharmaceutically acceptable salt" refers to those salts that are conventional acid-addition salts or base-addition salts that retain the biological effectiveness and properties of the compounds of the present invention, are formed from suitable non-toxic organic or inorganic acids or organic or inorganic bases and are suitable for use in, on or with the tissues of humans and lower animals without undue toxicity, irritation, allergic response and the like and are commensurate with a reasonable benefit/risk ratio.

The present invention is further explained by way of the following figures and examples, yet without being limited thereto.

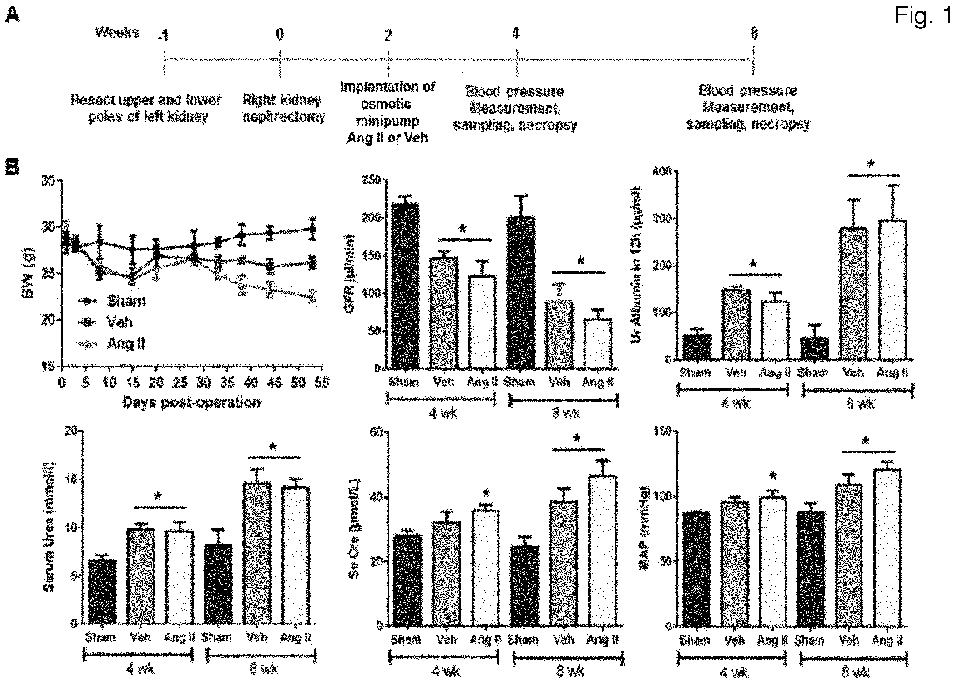

Figure 1 shows that Angiotensin II infusion does not significantly aggravate 5/6 nephrectomy-induced CKD progression in C57BL/6 6 mice on rescue diet. (A) Scheme of the 5/6 nephrectomy (5/6 Nx) protocol. Animals were included only if the ratio of resected left kidney/ removed right kidney was 0.55-0.72. (B) Body weight, glomerular filtration rate (GFR), urinary albumin, serum urea, serum creatinine, and mean arterial pressure in sham-operated (sham) and 5/6 Nx C57BL/6 mice on rescue diet (2% calcium, 1.25% phosphorus, 20% lactose), receiving additional subcutaneous infusions of angiotensin II (Ang II) or saline (vehicle, Veh) via osmotic mini-pumps for 4 and 8 weeks postsurgery. Each data point represents the mean ± SEM of 5 - 8 mice each. * denotes P < 0.05 vs. Sham, 1-way ANOVA followed by Student-Newman-Keuls test.

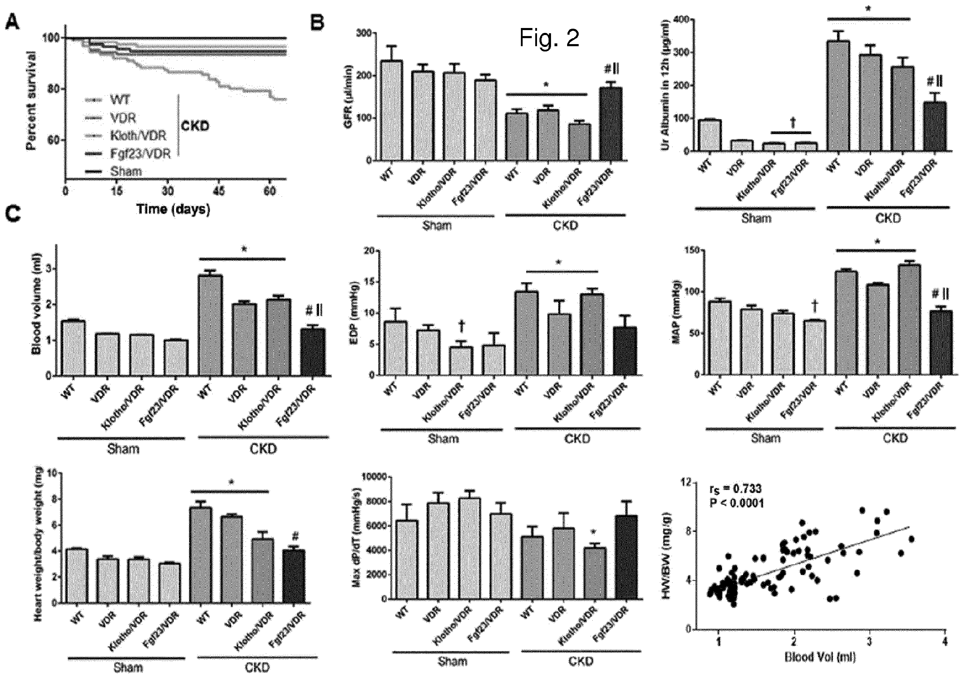

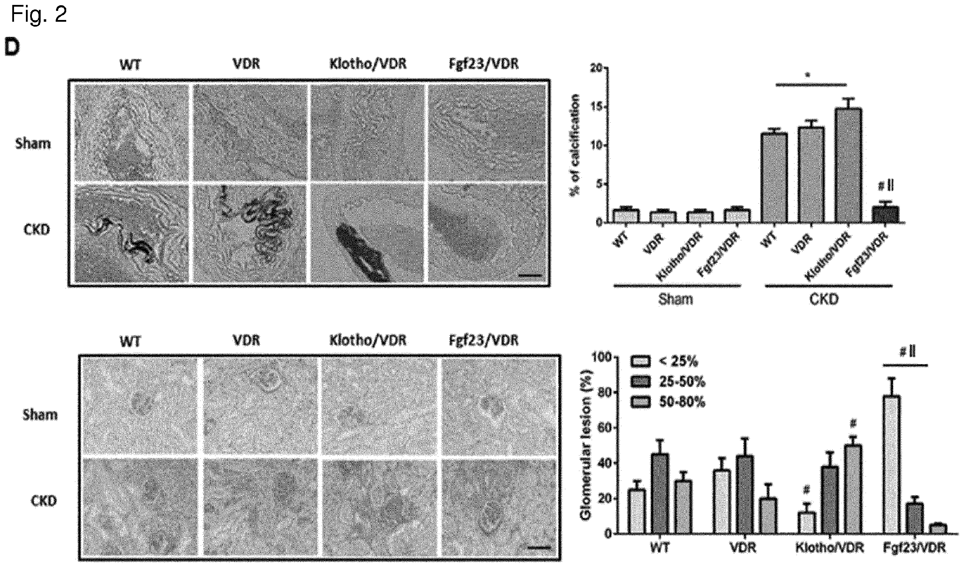

Figure 2 shows the effect of FGF23- or Klotho-deficiency on CKD-induced pathology in mice. (A) Survival curve and body weight, (B) glomerular filtration rate (GFR) and urinary albumin, (C) blood volume, left ventricular end-diastolic pressure (EDP), mean arterial pressure (MAP), heart-to-body weight ratio (HW/BW), cardiac contractility (dP/dT), and linear plot of HW/BW ratio on blood volume, (D) von Kossa staining of aortic sections, quantification of aortic calcification, representative images of kidney sections stained with periodic acid-Schiff (PAS) stain, and semi-quantitative assessment of glomerular injury as evidenced by mesangial expansion in PAS-stained sections in Sham-operated (Sham) and 5/6 Nx (CKD) WT, VDR, Klotho/VDR and Fgf23/VDR compound mutant mice, 8 weeks postsurgery. Each data point in bar graphs represents the mean ± SEM of 8 - 12 mice each. Original magnification x40 in D. * denotes P < 0.05 vs. Sham animals of the same genotype, t P < 0.05 vs. Sham VDR^{Δ/Δ} mutants, # P < 0.05 vs. CKD WT and VDR^{Δ/Δ} mice, □ P < 0.05 vs. CKD *Klotho*/VDR compound mutants, 1-way ANOVA followed by Student-Newman-Keuls test.

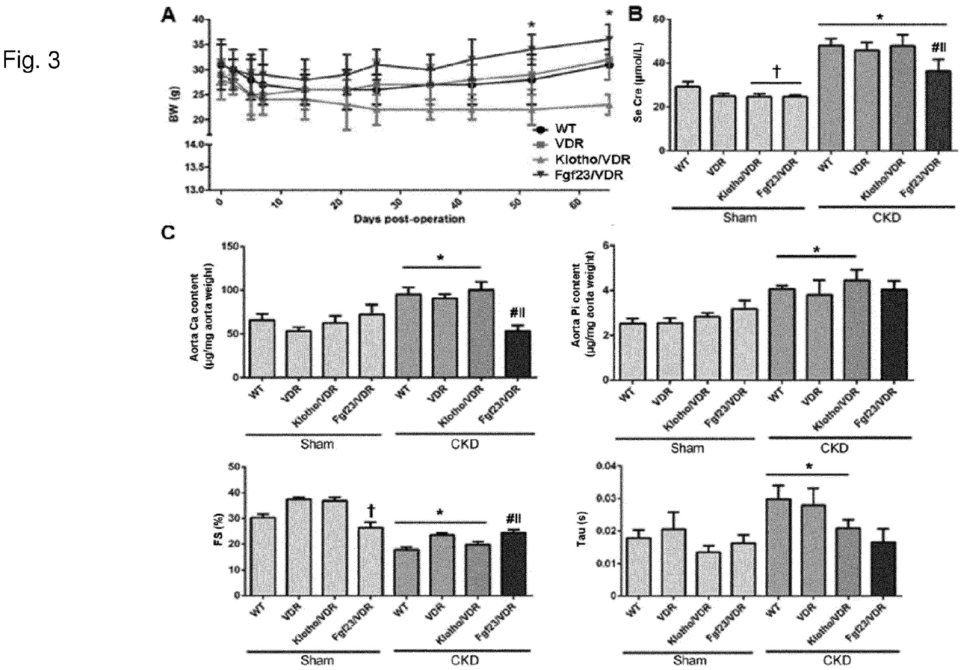

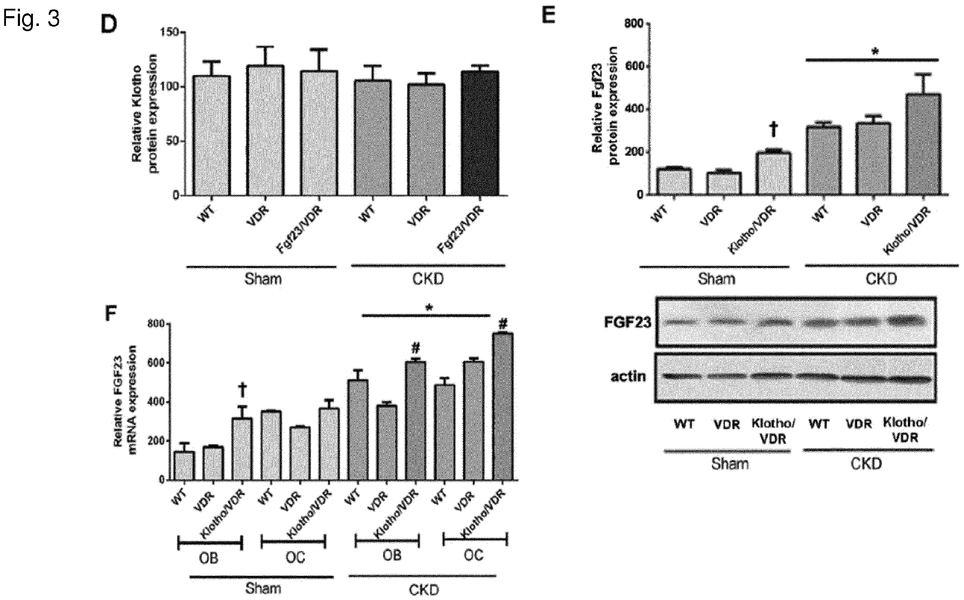

Figure 3 shows that lack of Fgf23 but not of Klotho ameliorates CKD-induced pathology. (A) Body weight, (B) serum creatinine, (C) aortic calcium and phosphate content assessed by biochemical analysis, cardiac fractional shortening (FS) and tau measured by echocardiography, (D) renal Klotho protein expression, (E) Fgf23 protein expression in total femur homogenates, and (F) quantification of Fgf23 mRNA expression in osteoblasts (OB) and osteocytes (OC) harvested by laser capture microdissection from femur cryo-sections in sham-operated (sham) and 5/6 Nx (CKD) WT, VDR, Klotho/VDR and Fgf23/VDR compound mutant mice, 8 weeks postsurgery. Each data point in A-B represents the mean ± SEM of 7 - 9 mice each. Each data point in D-E represents the mean ± SEM of 3 - 5 mice each. * denotes P < 0.05 vs. Sham animals of the same genotype, t P < 0.05 vs. Sham VDR^{Δ/Δ} mutants, # P < 0.05 vs. CKD WT and VDR^{Δ/Δ} mice, □ P < 0.05 vs. CKD Klotho/VDR compound mutants, 1-way ANOVA followed by Student-Newman-Keuls test.

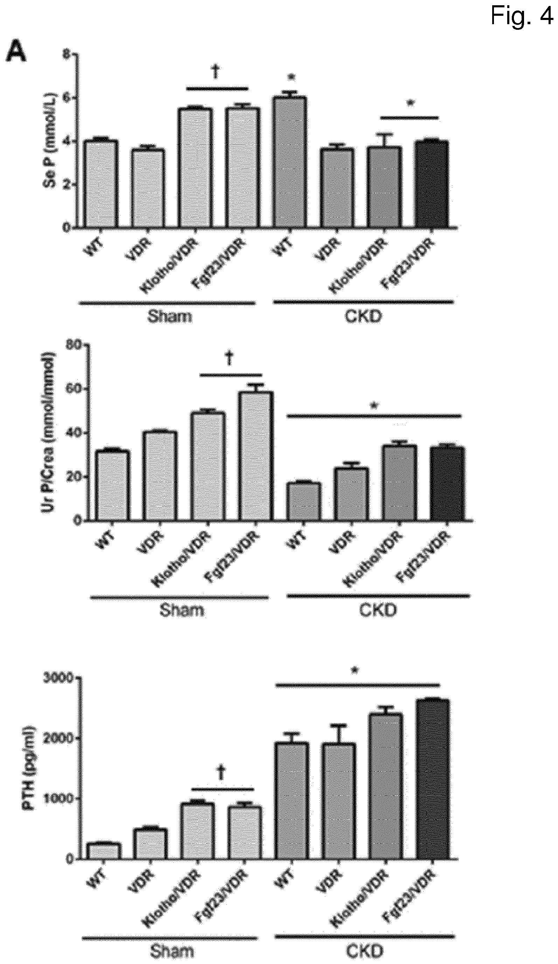

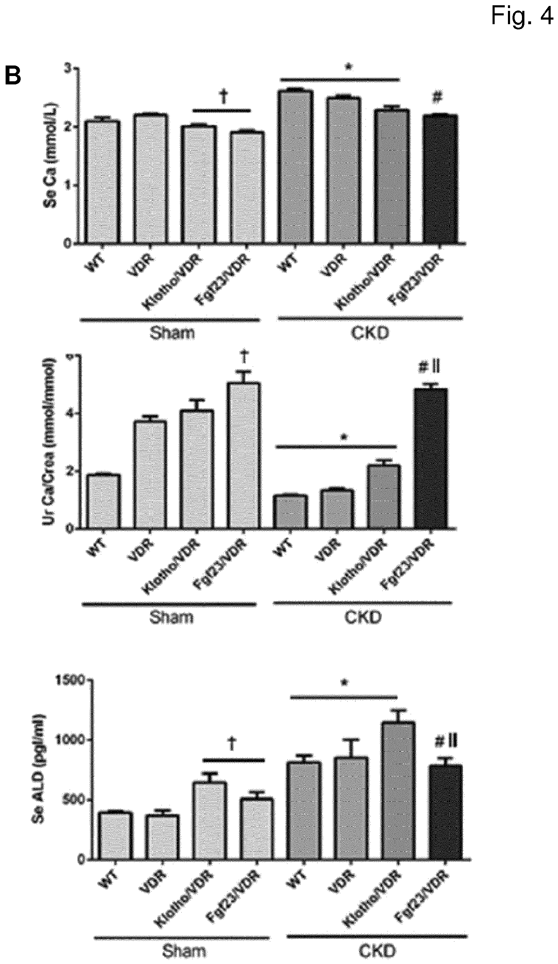

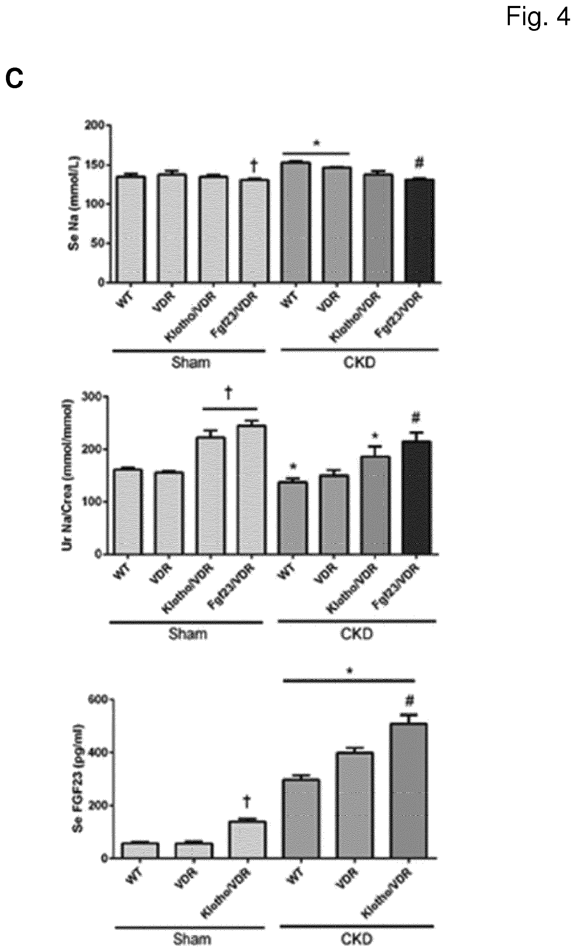

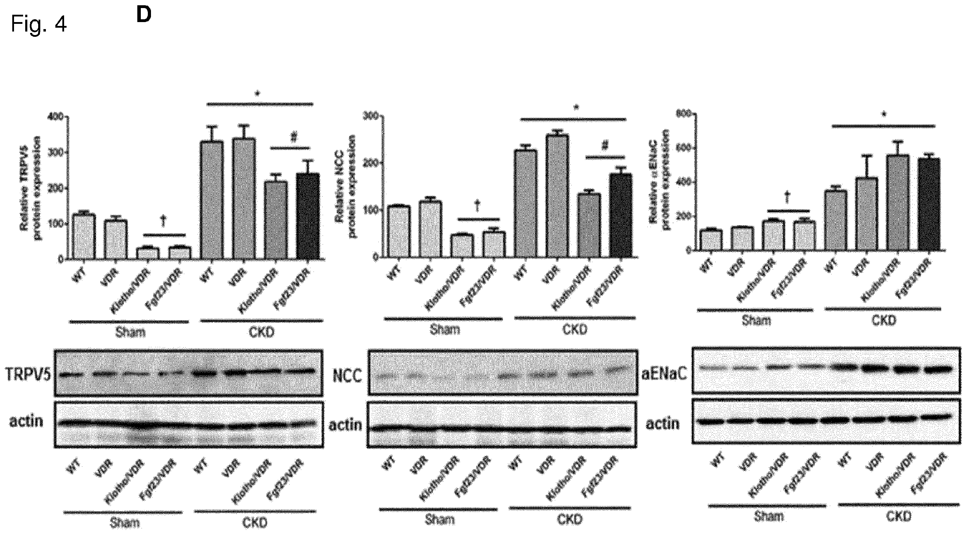

Figure 4 shows the effect of FGF23- or Klotho-deficiency on renal calcium and sodium retention in mice with CKD.(A) Serum phosphate, calcium and sodium, urinary calcium/creatinine (UrCa/Crea) excretion, urinary phosphate/creatinine (UrP/Crea) excretion, urinary sodium/creatinine (UrNa/Crea) excretion, serum PTH, aldosterone (ALD) and Fgf23 levels; (B) Relative TRPV5, NCC and αENaC protein expression in renal total membrane fractions or total kidney homogenates in Sham-operated (Sham) and 5/6 Nx (CKD) WT, VDR, Klotho/VDR and Fgf23/VDR compound mutant mice, 8 weeks postsurgery. Each data point in A represents the mean ± SEM of 8 - 12 mice each. Each data point in B represents the mean ± SEM of 4 - 5 mice each. * denotes P < 0.05 vs. Sham animals of the same genotype, t P < 0.05 vs. Sham VDR^{Δ/Δ} mutants, # P < 0.05 vs. CKD WT and VDR^{Δ/Δ} mutants, □ P < 0.05 vs. CKD Klotho/VDR compound mutants, 1-way ANOVA followed by Student-Newman-Keuls test.

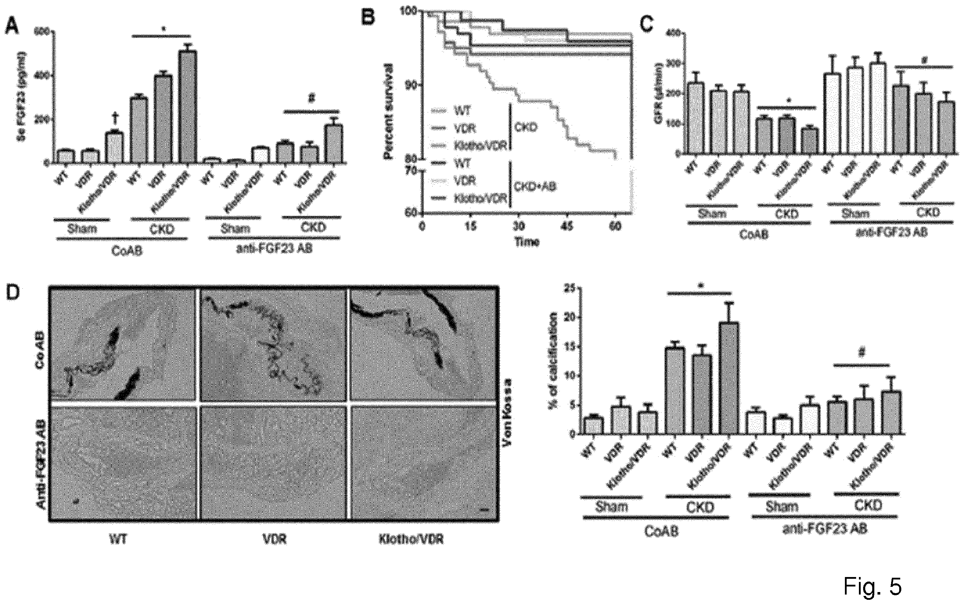

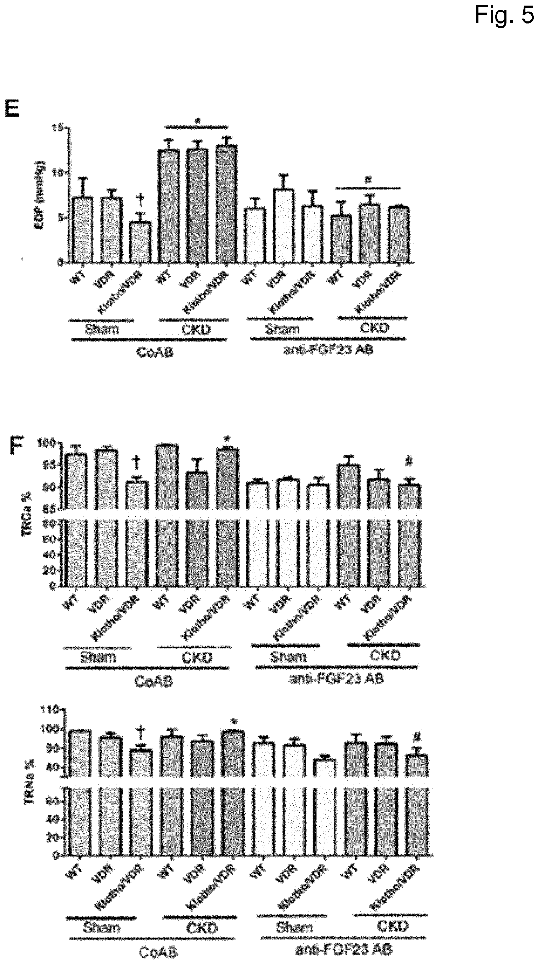

Figure 5 shows that pharmacological inhibition of FGF23 largely protects against the CKD-induced renal and cardiovascular pathology. (A) Serum Fgf23 levels, (B) survival curve, (C) GFR, (D) von Kossa staining of aortic sections and quantification of aortic calcification, (E) left ventricular end-diastolic pressure (EDP), (F) Percent tubular reabsorption of calcium (%TRCa) and of sodium (%TRNa) in Sham-operated (Sham) and 5/6 Nx (CKD) WT, VDR, and Klotho/VDR compound mutant mice treated for 8 weeks with control antibody (CoAB) or anti-FGF23 antibody (anti-FGF23 AB, 50 µg per mouse, two times per week), 8 weeks post-surgery. Each data point represents the mean ± SEM of 7 - 9 mice each. * denotes P < 0.05 vs. Sham animals of the same genotype treated with CoAB, t P < 0.05 vs. Sham VDR^{Δ/Δ} mutants treated with CoAB, # P < 0.05 vs. CKD mice of the same genotype treated with CoAB, 1-way ANOVA followed by Student-Newman-Keuls test.

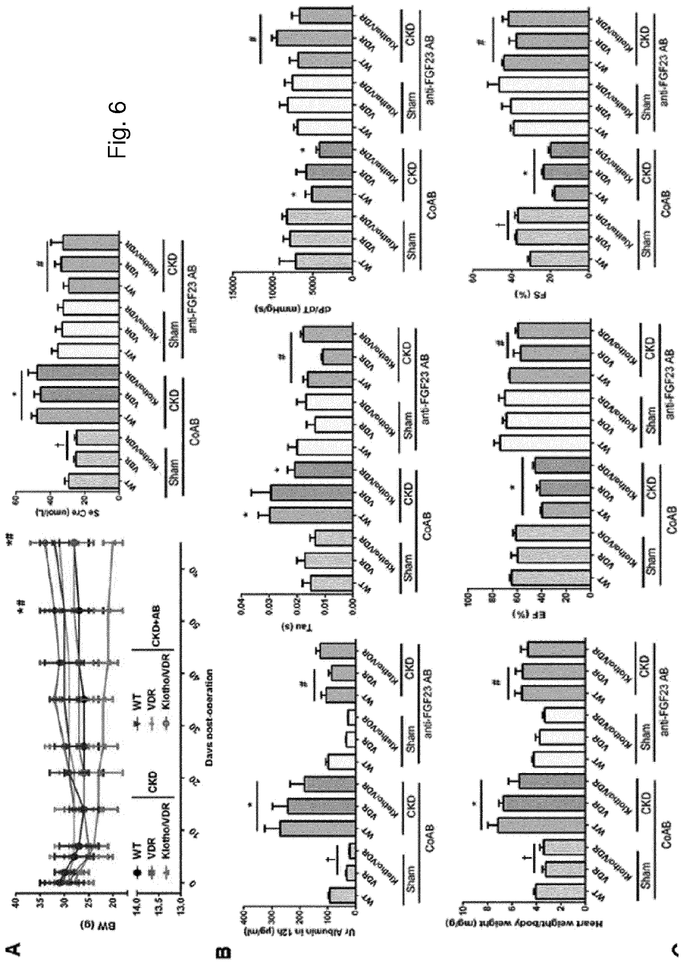

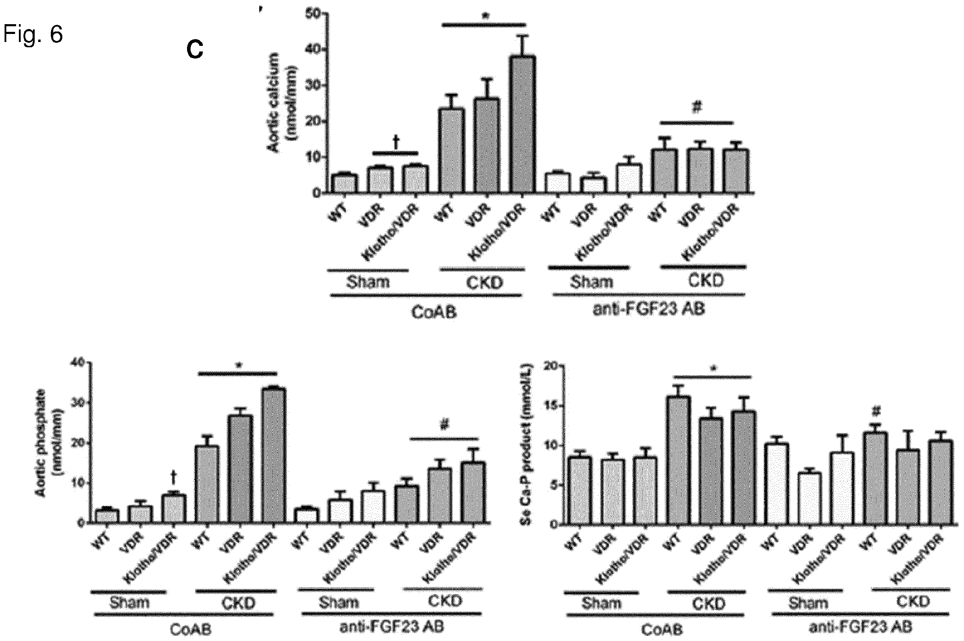

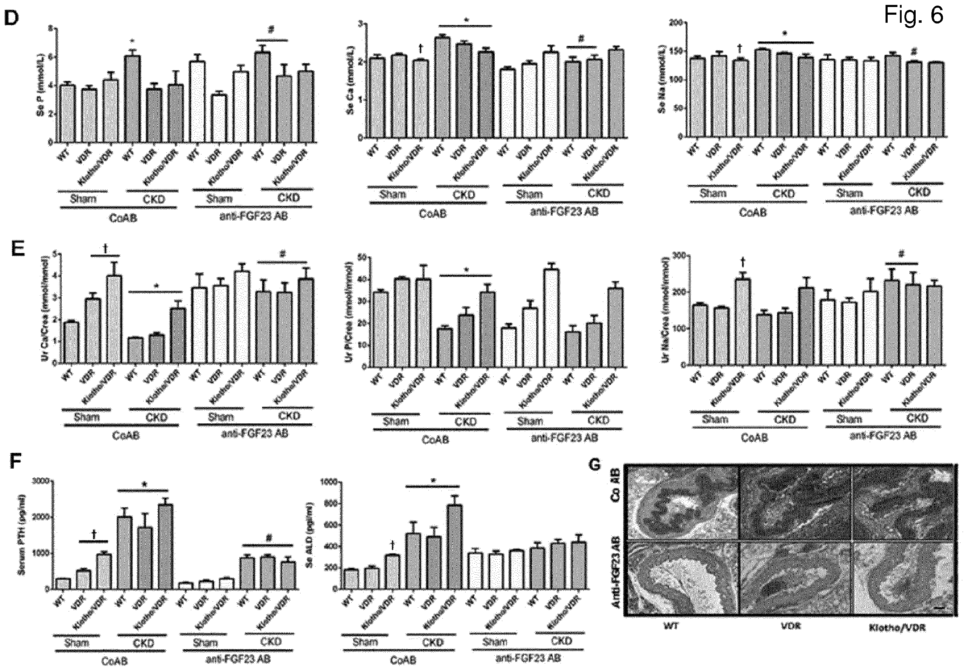

Figure 6 shows that anti-FGF23 antibody treatment largely protects against the CKD-induced renal and cardiovascular pathology. (A) Body weight and serum creatinine, (B) urinary albumin, tau, dP/dT, heart-to-body weight ratio, ejection fraction (EF) and fractioning shortening (FS), (C) aortic calcium and phosphate content and serum calcium-phosphate product, (D) serum phosphate, calcium and sodium, (E) urinary phosphate/creatinine (UrP/Crea) excretion, urinary calcium/creatinine (UrCa/Crea) excretion, and urinary sodium/creatinine (UrNa/Crea) excretion, (F) serum PTH and aldosterone (ALD), (G) Representative alizarin red-stained aortic sections (n = 3 - 5 mice each) in Sham-operated (Sham) and 5/6 Nx (CKD) WT, VDR, and Klotho/VDR compound mutant mice treated for 8 weeks with control antibody (Co-AB) or anti-FGF23 antibody (anti-FGF23 AB, 50 µg per mouse, two times per week), 8 weeks post-surgery. Each data point in A-F represents the mean ± SEM of 7 - 12 mice each. * denotes P < 0.05 vs. Sham animals of the same genotype treated with CoAB, t P < 0.05 vs. Sham VDRΔ/Δ mutants treated with CoAB, # P < 0.05 vs. CKD mice of the same genotype treated with CoAB, 1-way ANOVA followed by Student-Newman-Keuls test.

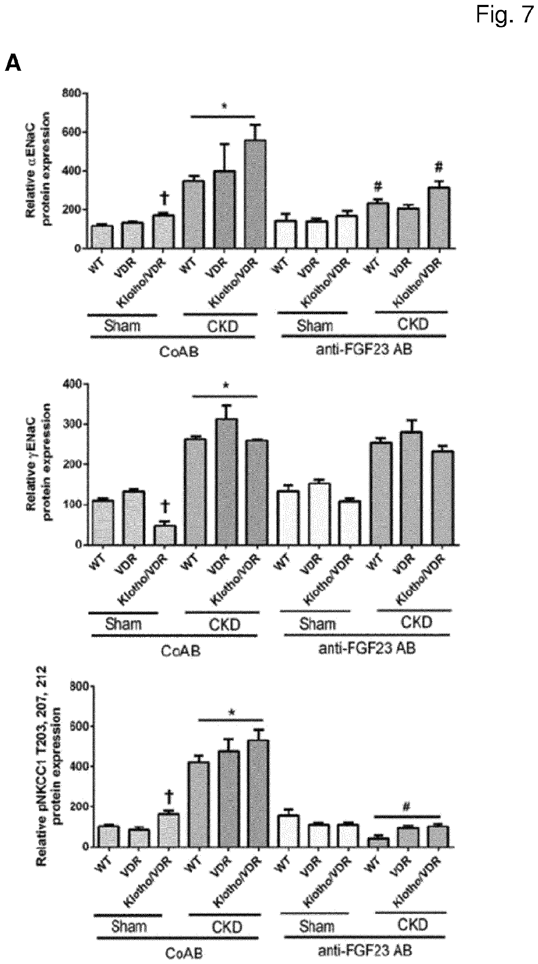

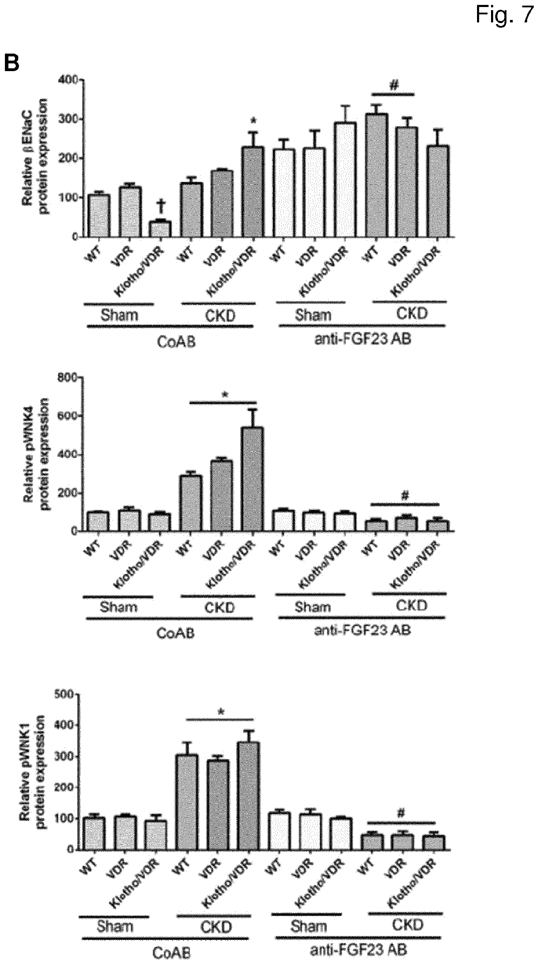

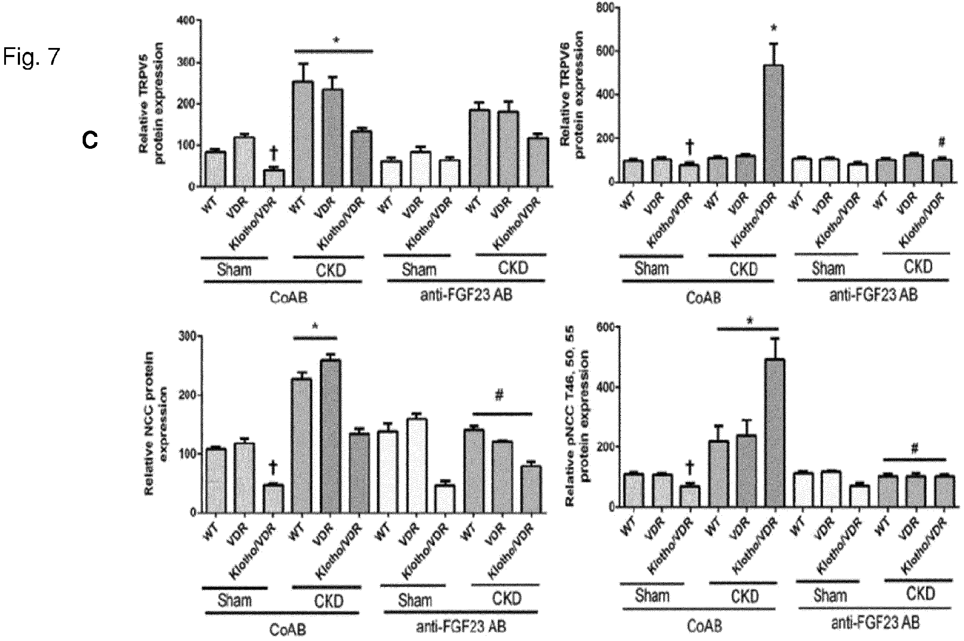

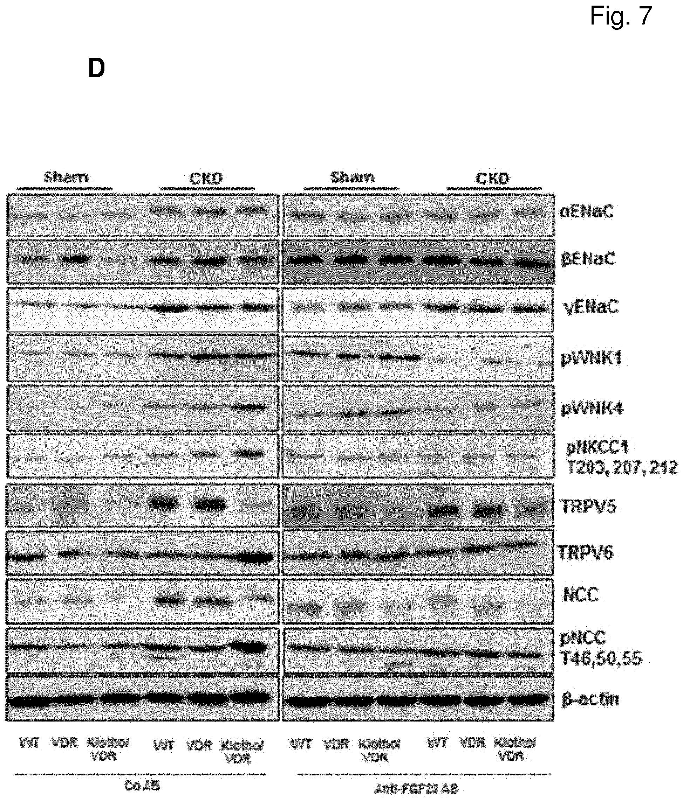

Figure 7 shows that anti-FGF23 antibody treatment suppresses WNK-mediated Klotho-dependent and -independent activation of renal calcium- and sodium-transporting molecules in CKD mice. Quantification and original Western blotting images of αENaC, βENaC, γENaC, phospho-NKCC1 (pNKCC1 T203, 207, 212), phospho-WNK4 (pWNK4), phospho-WNK1 (pWNK1), TRPV5, TRPV6, NCC and phospho-NCC (pNCC T45, 50, 55) protein expression in renal total homogenates or in renal total membrane fractions (TRPV5 and TRPV6) in Sham-operated (Sham) and 5/6 Nx (CKD) WT, VDR, and Klotho/VDR compound mutant mice treated for 8 weeks with control antibody (CoAB) or anti-FGF23 antibody (anti-FGF23 AB, 50 µg per mouse, two times per week), 8 weeks post-surgery. Each data point represents the mean ± SEM of 4 -5 mice each. * denotes P < 0.05 vs. Sham animals of the same genotype treated with CoAB, † P < 0.05 vs. Sham VDRΔ/Δ mutants treated with CoAB, # P < 0.05 vs. CKD mice of the same genotype treated with CoAB, 1-way ANOVA followed by Student-Newman-Keuls test.

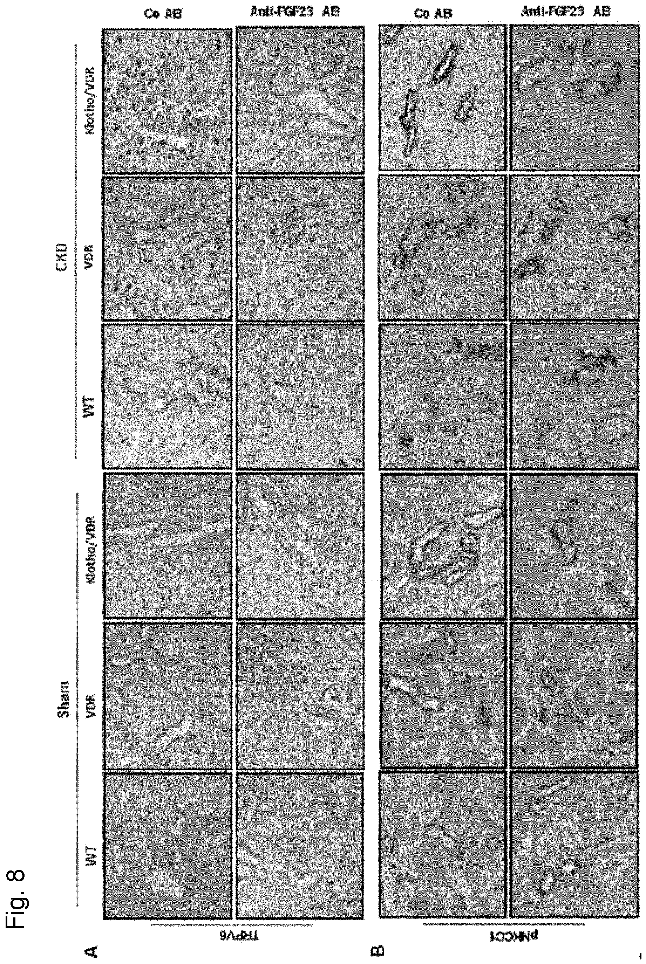

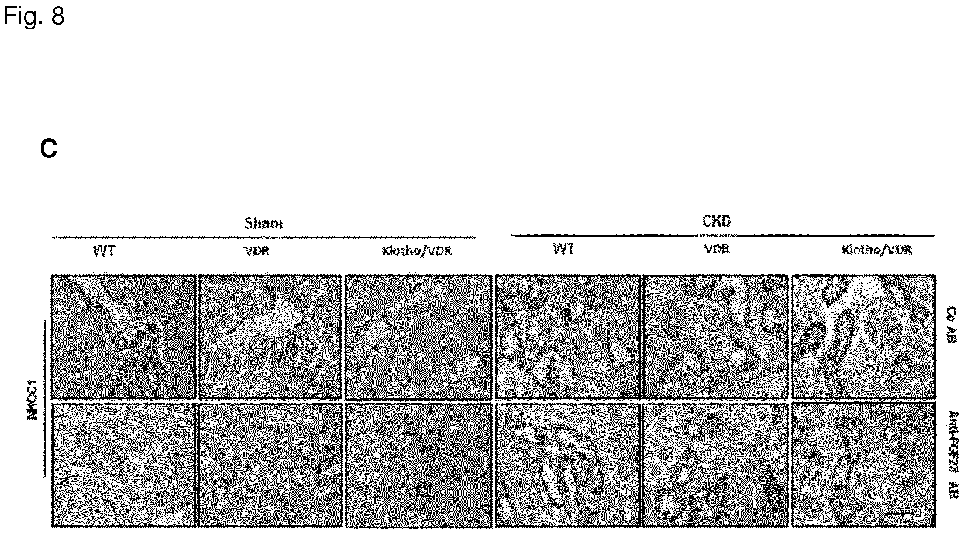

Figure 8 shows that treatment with anti-FGF23 antibody decreases distal renal tubular TRPV6 and pNKCC1 protein expression in Klotho/VDR CKD mice. (A) Representative images of TRPV6, (B) pNKCC1, and (C) NKCC1 immunohistochemistry in Sham-operated (Sham) and 5/6 Nx (CKD) WT, VDR, and Klotho/VDR compound mutant mice treated for 8 weeks with control antibody (CoAB) or anti-FGF23 antibody (anti-FGF23 AB, 50 µg per mouse, two times per week), 8 weeks post-surgery. Original magnification x40. Scale bar, 50 µm.

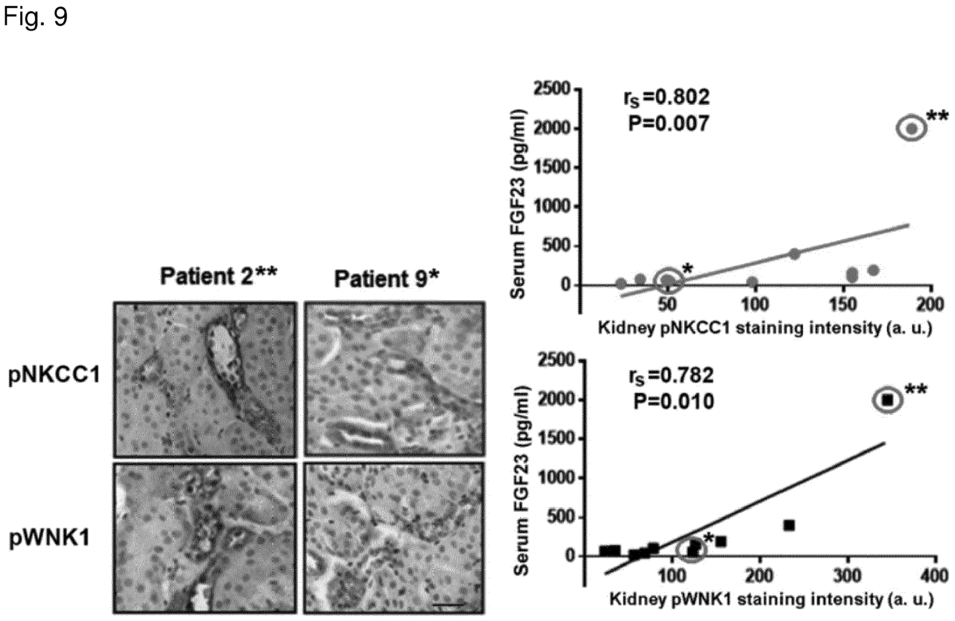

Figure 9 shows that serum intact FGF23 levels are associated with WNK activation in the kidney of human CKD patients. Representative immunohistochemical images of Patient 2 (**) and Patient 9 (*), and association between serum intact FGF23 with protein expression of phospho-NKCC1 T212, 217 (pNKCC1) and phospho-WNK1 S382 (pWNK1) quantified by immunohistochemistry in renal biopsies of 10 patients with CKD stages 2 - 5, and expressed as average staining intensity (a.u., arbitrary units). Insets show Spearman rank correlation coefficients. Bar = 50 µm.

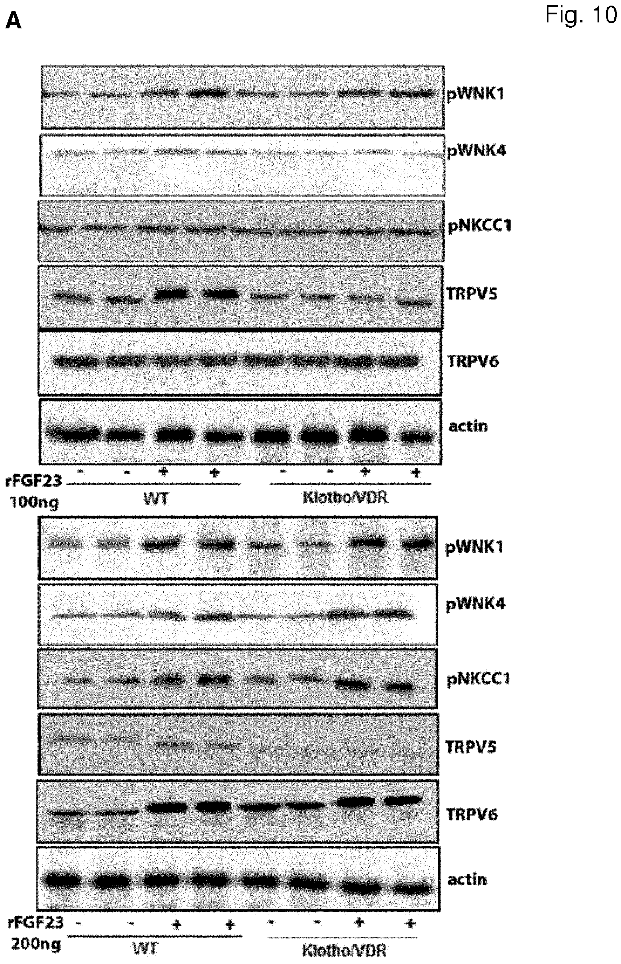

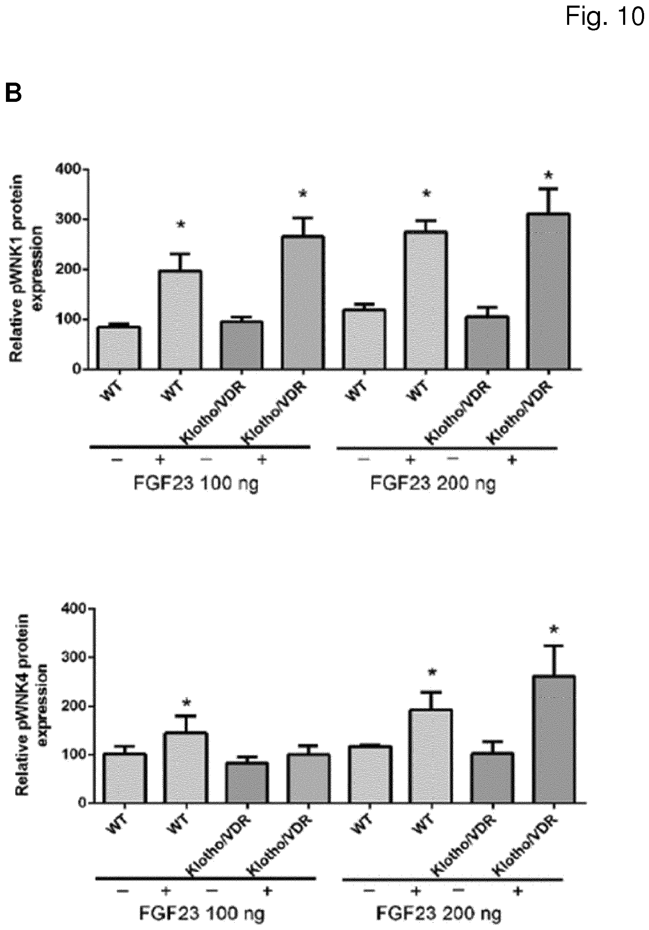

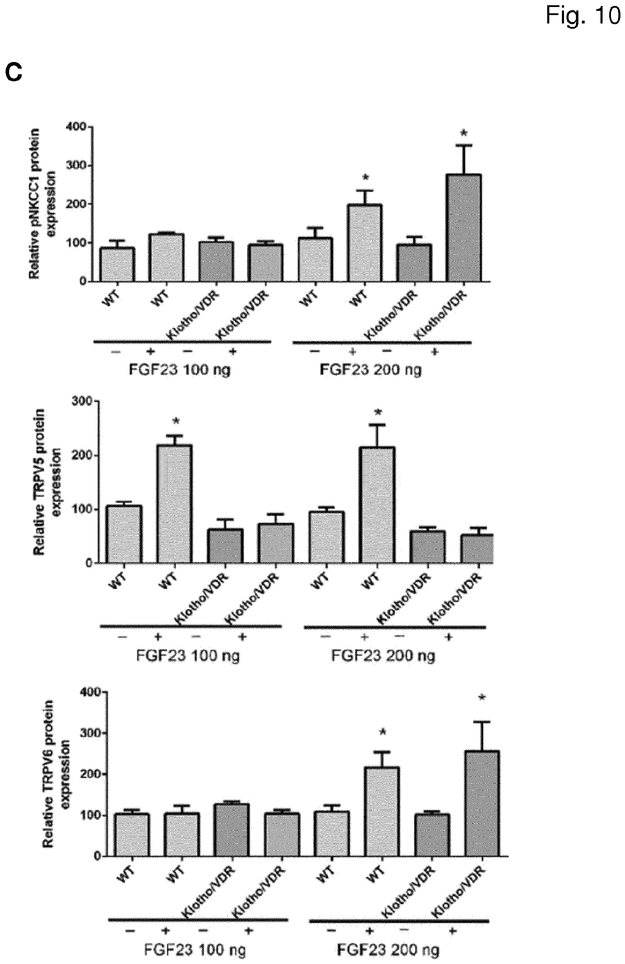

Figure 10 shows that very high concentrations of FGF23 activate WNK signalling in a Klotho-independent manner in kidney slices ex vivo. Quantification and original Western blotting images of phospho-WNK1 (pWNK1), phospho-WNK4 (pWNK4), phospho-NKCC1 (pNKCC1 T203, 207, 212), TRPV5, and TRPV6 protein expression in homogenates of 200-µm-thick live kidney slices from 3-month-old WT and Klotho/VDR mutant mice treated ex vivo for 2 h with vehicle or rFGF23 (100 ng/ml or 200 ng/ml). Data represent mean ± SEM of of 3 mice each. * denotes P < 0.05 vs. vehicle-treated slices of the same genotype.

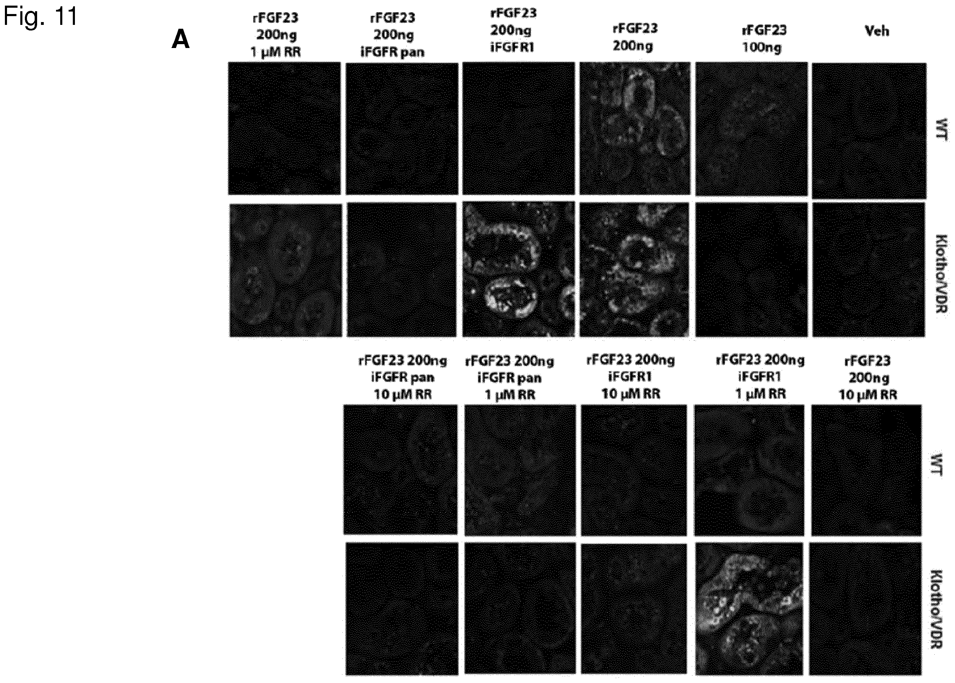

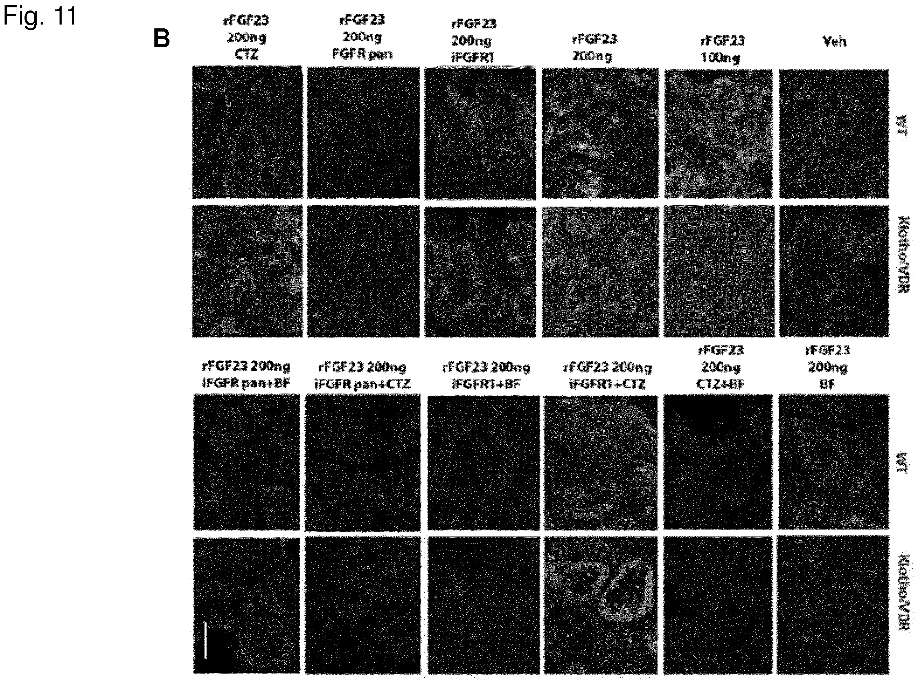

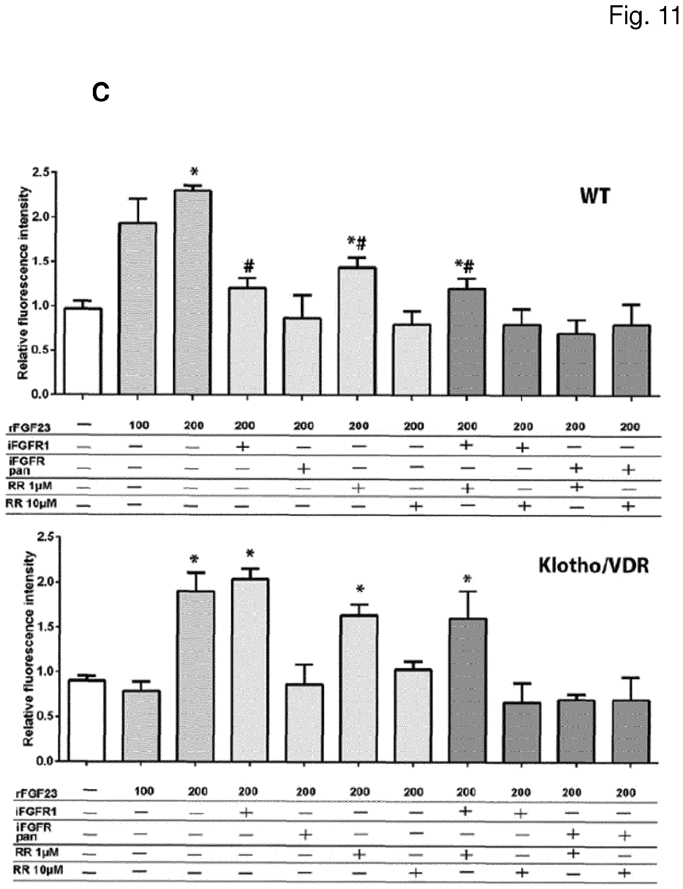

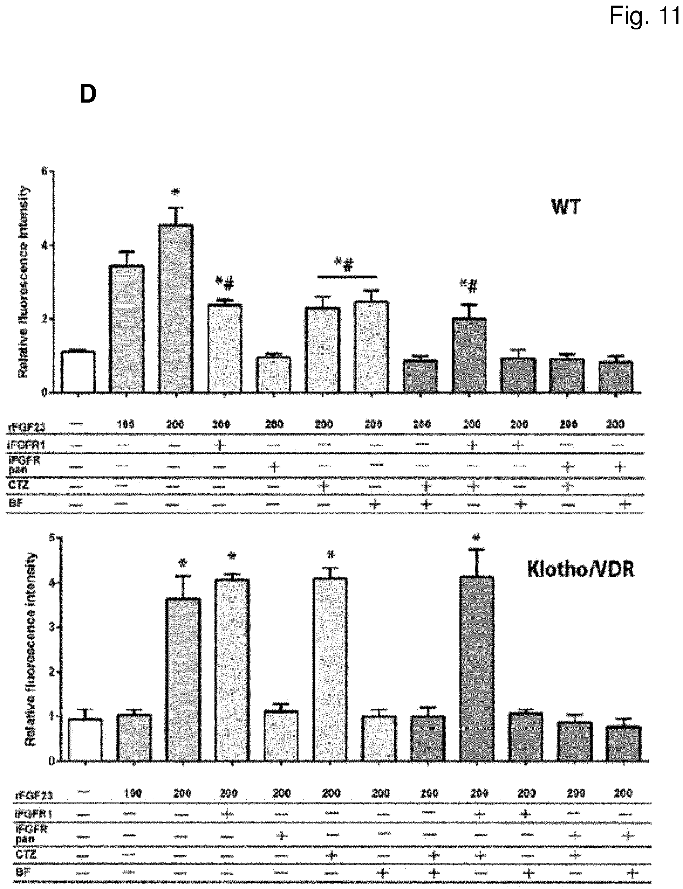

Figure 11 shows that very high concentrations of FGF23 stimulate distal tubular calcium and sodium transport in kidney slices by Klotho- and FGFR1-independent activation of NKCC1 and TRPV6 channels. (A) Original images and quantification of intracellular Ca2+ levels in renal distal tubules in Fluo-4-loaded, 200-µm-thick, live kidney slices from 3-month-old WT and Klotho/VDR mutant mice treated for 2 h with vehicle, rFGF23 (100 ng/ml or 200 ng/ml) alone or combined with a FGFR1 inhibitor (iFGFR1) or a pan FGFR inhibitor (iFGFR pan). Ruthenium red (RR) was used as TRPV inhibitor, 1 µM RR was used for TRPV5 inhibition and 10 µM RR was used for TRPV6 inhibition. (B) Original images and quantification of intracellular Na+ levels in renal distal tubules in SBFI-loaded, 200-µm-thick, live kidney slices from 3-month-old WT and Klotho/VDR mutant mice treated for 2 h with vehicle, rFGF23 (100 ng/ml or 200 ng/ml), alone or in combination with a FGFR1 inhibitor (iFGFR1) or a pan FGFR inhibitor (iFGFR pan). Chlorothiazide (CTZ, 10 µM) was used as NCC inhibitor and bumetanide (BF, 15 µM) was used as NKCC1 inhibitor. Fluorescence intensity in A and B was quantified in 4-6 regions of interest per image, sample, and time point from 2-3 independent experiments. Data represent mean ± SEM. * denotes P < 0.05 vs. vehicle-treated, # P < 0.05 vs. slices treated with 200 ng/ml rFGF23 alone.

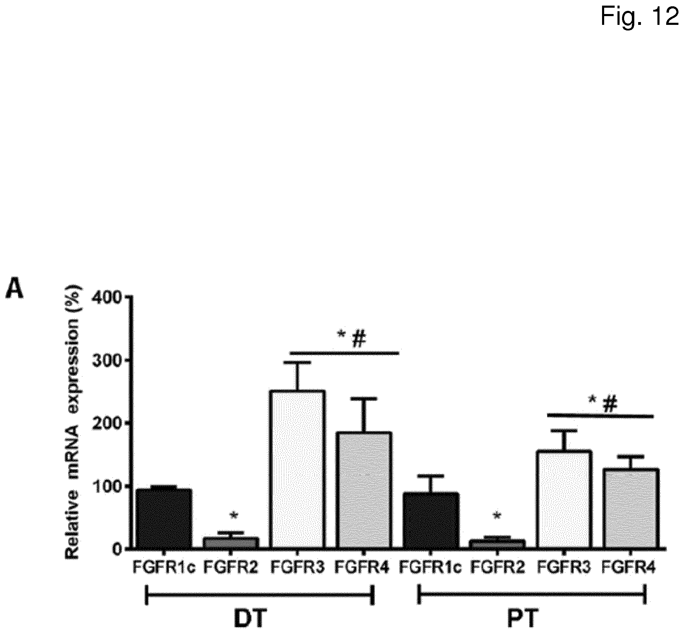

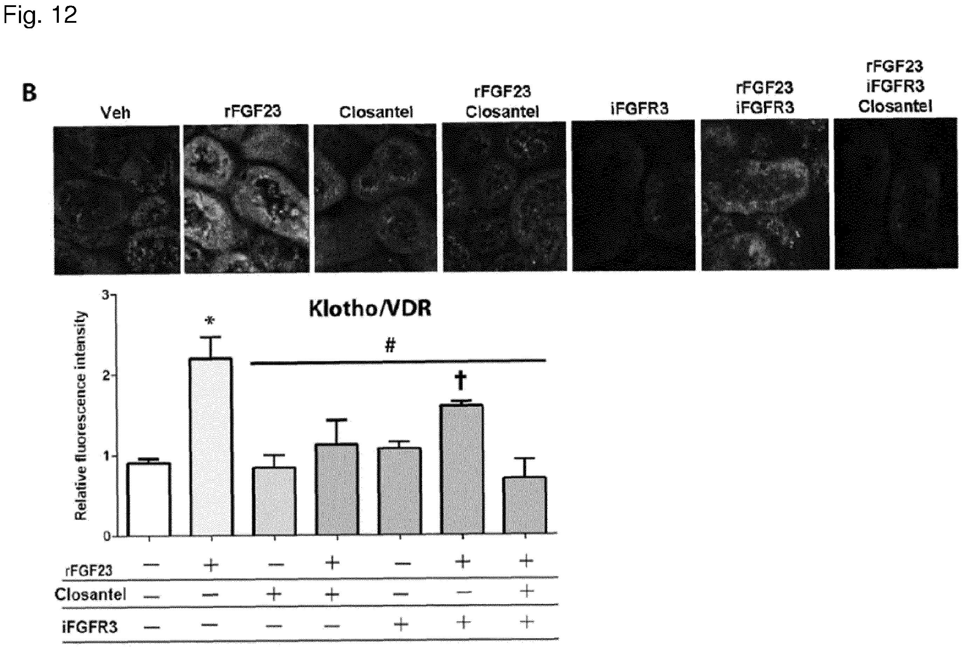

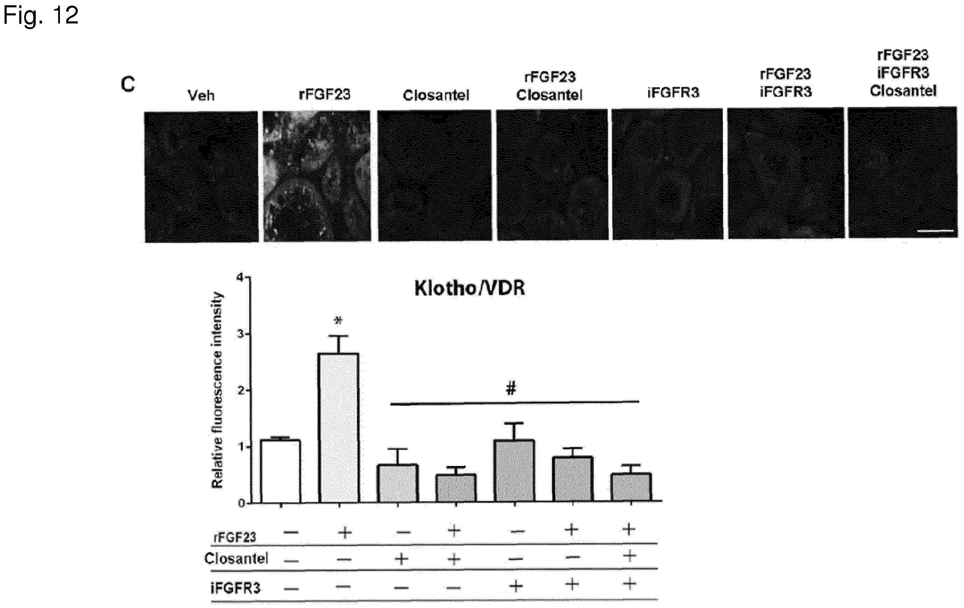

Figure 12 shows renal FGFR mRNA expression and effects of FGFR1/3 and/or WNK signalling inhibition on distal tubular FGF23-mediated calcium and sodium uptake in kidney slices of Klotho/VDR mutants. (A) FGFR1c, FGFR2, FGFR3 and FGFR4 mRNA expression levels in distal (DT) and proximal renal tubules (PT) of WT mice harvested by laser capture microdissection. Data represent mean ± SEM of 4 animals. * denotes P < 0.05 vs. FGFR1c mRNA expression, # P < 0.05 vs. FGFR2 mRNA expression, 1-way ANOVA followed by Student-Newman-Keuls test. (B) Original images and quantification of intracellular Ca²⁺ levels in renal distal tubules in Fluo-4-loaded, 200-µm-thick, live kidney slices of 3-month-old Klotho/VDR mutant mice treated for 2 h with vehicle (PBS), rFGF23 (200 ng/ml) alone or in combination with the WNK signalling inhibitor closantel or a FGFR1/3 inhibitor (iFGFR3). (C) Original images and quantification of intracellular Na+ levels in renal distal tubules in SBFI-loaded, 200-µm-thick, live kidney slices of 3-month-old Klotho/VDR mutant mice treated for 2 h with vehicle, rFGF23 (200 ng/ml) alone or combined with the WNK signalling inhibitor closantel or a FGFR1/3 inhibitor (iFGFR3). Fluorescence intensity was quantified in 4-6 regions of interest per image, sample, and time point from 2-3 independent experiments. Data represent mean ± SEM.* denotes P < 0.05 vs. vehicle-treated, # P < 0.05 vs. slices treated with 200 ng/ml rFGF23 alone, † P < 0.05 vs. slices treated with iFGFR3 alone, 1-way ANOVA followed by Student-Newman-Keuls test.

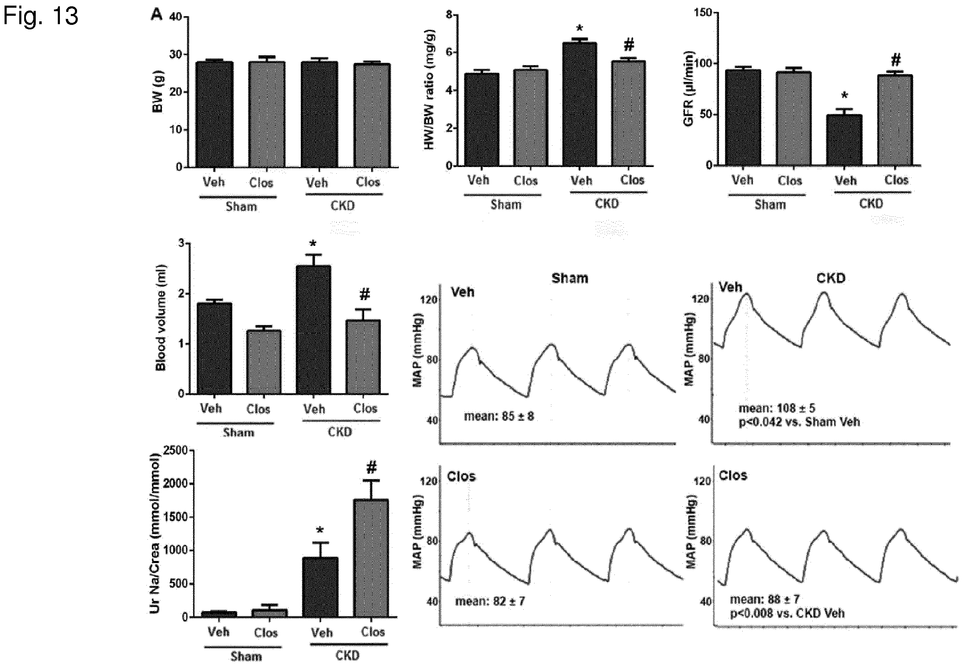

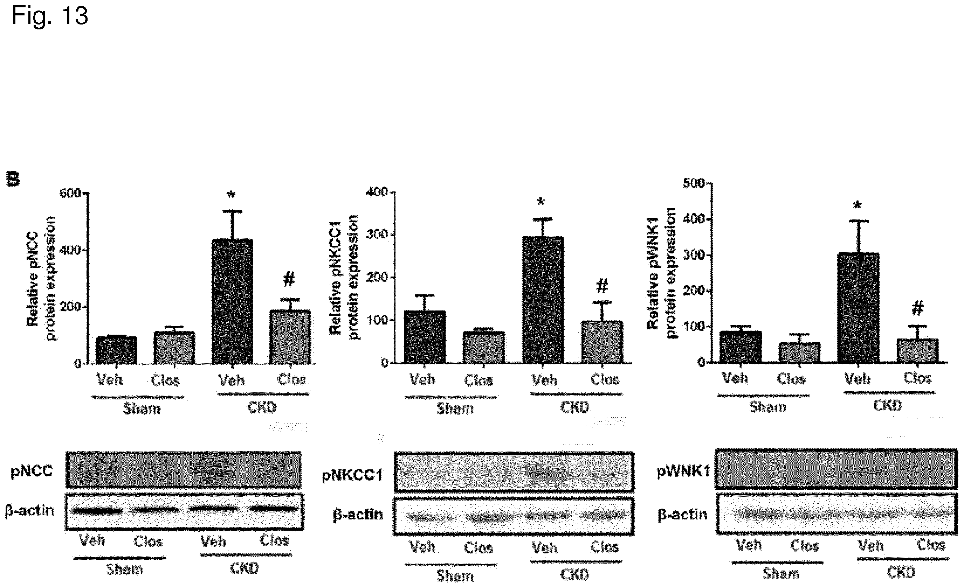

Figure 13 shows that the WNK signalling inhibitor closantel largely protects against disease progression in CKD mice. (A) Body weight, HW/BW ratio, GFR, blood volume, mean arterial pressure (MAP), urinary sodium/creatinine (UrNa/Crea) excretion, and (B) quantification and original Western blotting images of phospho-NCC (pNCC), phospho-NKCC1 (pNKCC1), and phospho-WNK1 (pWNK1) protein expression in renal total homogenates of Sham-operated (Sham) and 5/6 Nx (CKD) WT mice on rescue diet treated over 8 weeks once a week with closantel orally via the diet, 8 weeks postsurgery. Each data point represents the mean ± SEM of 7 - 9 mice each. * denotes P < 0.05 vs. Sham + vehicle (Veh), # P < 0.05 vs. CKD + Veh, 1-way ANOVA followed by Student-Newman-Keuls test.

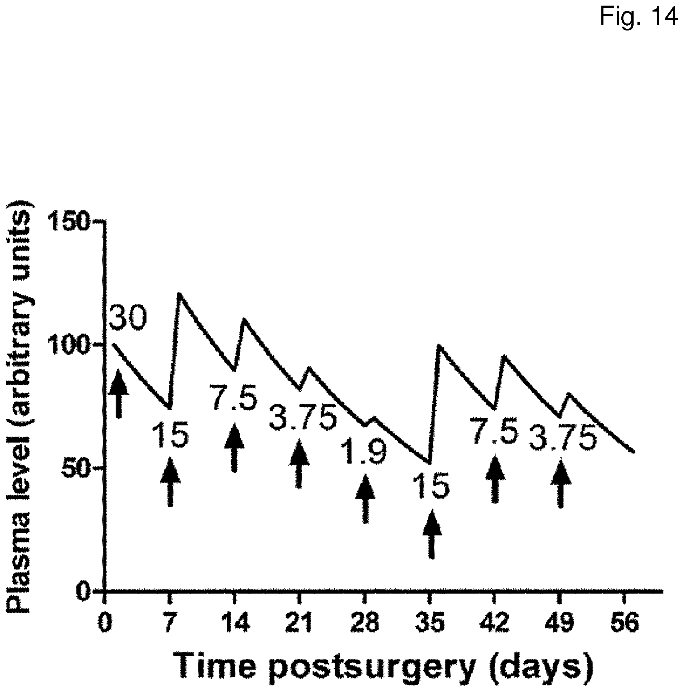

Figure 14 shows the calculated plasma levels (arbitrary units) after oral administration of closantel at varying doses once a week via the diet. The plasma level after the loading dose (5 mg/kg body weight, corresponding to 30 mg/kg diet) given on Day 1 was set to 100. Closantel-containing food at the concentrations (mg/kg diet) shown in the graph were administered on Days 1, 8, 15, 22, 29, 36, 43, and 50 (arrows) after Sham surgery or 5/6-Nx.

### EXAMPLES

### Materials and methods:

### Human study

The human study was approved by the Ethical Committee of the Medical University of Vienna on Nov 17, 2016 (#2006/2016), and each patient provided written consent for the use of the biopsy and plasma for this specific study. We prospectively collected kidney biopsies and serum from ten patients with various stages of chronic kidney disease (CKD stage 2 to 5). Serum was aliquoted and biopsy specimen split in half for paraffin and cryo-embedding. Five-µm-thick paraffin sections were cut on a microtome and further processed for anti-pWNK1, anti-pNCC, and anti-pNKCC1 immunohistochemistry. Each patient received a full set of routine lab work up few days before the biopsy including electrolytes, creatinine, BUN, iPTH, 1,25(OH)2D, and intact FGF23 (ELISA, Kainos) measurements.

### Animals

All animal studies were approved by the Ethical Committee of the University of Veterinary Medicine, Vienna and by the Austrian Federal Ministry of Science and Research and were undertaken in strict accordance with prevailing guidelines for animal care. All efforts were made to minimize animal suffering. Heterozygous VDR^{+/Δ} mutant mice (Erben R G et al., Mol. Endocrinol., 2002. 16, 1524-1537) and heterozygous Fgf23^{+/-} (Sitara D et al., Matrix Biol., 2004. 23, 421-432) and Klotho^{+/-} (Lexicon Genetics, Mutant Mouse Regional Resource Centers, University of California, Davis) mice were mated to generate double heterozygous animals. The VDR, Fgf23 and Klotho mutant mice used for the matings had been backcrossed to C57BL/6 genetic background for 10 generations. The double heterozygous offspring from these matings were intercrossed to generate wild-type (WT), Fgf23^{-/-}, VDR^{Δ/Δ}, Fgf23^{-/-} /VDR^{Δ/Δ} and Klotho^{-/-}/VDR^{Δ/Δ} mice. Genotyping of the mice was performed by multiplex PCR using genomic DNA extracted from the tail as described in Hesse M et al. (Matrix Biol., 2007. 26, 75-84) and Anour R et al. (PLoS. One., 2012. 7, e31376). The mice were kept at 24°C with a 12 h/12 h light/dark cycle, and were allowed free access to a normal rodent chow or the rescue diet and tap water. The rescue diet (Ssniff, Soest, Germany) containing 2.0% calcium, 1.25% phosphorus, 20% lactose and 600 IU vitamin D/kg was fed starting from 16 days of age. This diet has been shown to normalize mineral homeostasis in VDR-ablated mice (Erben R G et al., Mol. Endocrinol., 2002. 16, 1524-1537). All experiments were performed on 3-month-old male offspring of double heterozygous x double heterozygous matings. Urine was collected in metabolic cages before necropsy. At necropsy, mice were exsanguinated from the abdominal V. cava under general anesthesia (ketamine/xylazine, 100/6 mg/kg i.p.) for serum collection.

### Serum and urine biochemistry

Serum and urinary calcium, phosphorus, albumin and creatinine were analyzed on a Cobas c111 analyzer (Roche). Renal tubular reabsorption of calcium (TRCa) and sodium (TRNa) were calculated according to the formula %TRCa = [1-(UrCa*SeCrea)/(SeCa*UrCrea)]*100 and %TRNa = [1-(UrNa*SeCrea)/(SeNa*UrCrea)]*100 (Ur, urinary; Se, serum; Crea, creatinine; Ca, calcium; Na, sodium). Serum intact PTH, serum aldosterone (ALD), and serum intact FGF23 were determined by ELISA (Immutopics, NovaTec, and Kainos, respectively).

### 5/6 nephrectomy (5/6 Nx) model

The 5/6 nephrectomy (5/6 Nx) was performed in two stages (Leelahavanichkul A et al., Kidney Int. 78, 2010. 1136-1153) under isoflurane anesthesia (Fig. 1A). Pain was managed by s.c. injections of buprenorphine, metamizol and meloxicam. Additionally, the animals were treated for 3 days postsurgery with the analgesic piritramid via the drinking water, starting 6 h postsurgery. At the first stage (wk -1), the left kidney was decapsulated to avoid ureter and adrenal damage, and the upper and lower poles were partially resected via a left flank incision. Bleeding was controlled by microfibrillar collagen hemostasis (Avitene, Davol, Cranston, RI). The upper and lower poles were weighed. One week later, the entire right kidney was removed via a right flank incision and weighed. Animals with insufficient kidney resection as determined by a ratio of < 0.55 for the removed left kidney weight to the removed right kidney weight were euthanized. In the Sham surgery control mice, a left flank incision was performed at step 1, both poles of kidney were identified, then the flank incision was closed; a subsequent right flank incision was performed at step 2; the right renal artery was identified, then the flank incision was closed. After each flank incision, muscles and skin were repositioned and sewed. Mortality was tracked throughout the study. Necropsy was not performed on animals that died early.

### Angiotensin II administration

Angiotensin II (Ang II) 0.75 µg/kg/min (Sigma) in physiological saline or vehicle was infused by subcutaneous osmotic mini-pumps (Alzet model 1004, Cupertino, CA) (Leelahavanichkul A et al., Kidney Int. 78, 2010. 1136-1153). The osmotic minipumps were inserted subcutaneously at the back under isoflurane anesthesia at 5 days after right NX or Sham surgery (Fig. 1A).

### Neutralizing anti-FGF23 antibody administration

Neutralizing anti-FGF23 antibody (anti-FGF23Ab, 50 µg per mouse) or an isotype control Ab (Co Ab, 50 µg per mouse) were administered 2 times per week for 8 weeks beginning on day 0 (baseline) via intravenous administration into a tail vein.

### Closantel administration

For this experiment, WT Sham and 5/6-Nx mice were fed powdered rescue diet throughout the study. Closantel (Abcam) was dissolved in 100% ethanol (vehicle), and mixed into the moistened diet. To achieve a loading dose of 5 mg/kg body weight, the drug was mixed into the food at an initial concentration of 30 mg/kg diet. The concentration in the food was calculated based on a mean food consumption of 8.5 g per day per mouse, and an approximate bioavailability of 50%. To maintain constant plasma levels, closantel was administered once a week using the dosing regimen shown in Fig. 14. The model used to calculate the theoretical plasma levels was based on the assumption of a plasma half-life of 14 days (WHO Food Additives Series 27, http://www.inchem.org/documents/jecfa/jecmono/v27je02.htm). All mice were killed 8 weeks postsurgery.

### Transthoracic Doppler echocardiography (ECG)

Left ventricular (LV) function was non-invasively assessed in all mice prior to necropsy under anesthesia with isofluorane by ECG, using a 14MHz linear transducer (Siemens S2000). Short axis m-mode recordings of LV were obtained from a left parasternal acoustic window. LV dimensions in systole and diastole were measured at the largest diameter of the ventricle at the level of the papillary muscles, and systolic parameters such as fractional shortening (FS) and ejection fraction (EF) were calculated. Diastolic LV function was evaluated using the pulsed-wave Doppler recording of trans-mitral blood flow velocities in the apical 4-chamber view. A minimum of 4 cardiac cycles were averaged for measurement of each parameter. Total duration of examination did not exceed 30 min.

### Central arterial pressure measurements and cardiac catheterization

Central arterial pressure and cardiac hemodynamic parameters were assessed using a SPR-671NR pressure catheter (1.4 French; Millar Instruments). Mice were anesthetized with 1.5% isoflurane in oxygen. The catheter was inserted into the ascending aorta for measurement of central arterial pressure. Parameters of LV function (i.e., maximum rate of the pressure rise, contractility index, end-diastolic pressure, and isovolumetric relaxation time) were measured by placing the catheter into the left ventricle. All pressure recordings were performed at the same anesthesia level. Hemodynamic measurements were recorded over 5 min, and traces were analyzed using LabChartPro software and a blood pressure (BP) module. After pressure measurements, all animals were euthanized.

### Blood volume measurements

The blood volume was determined from plasma volume and hematocrit as described in Lee H B & Blaufox M D (J. Nucl. Med., 1985. 26, 72-76). Plasma volume was determined by Evans blue dye dilution (Barron W M et al., J. Clin. Invest, 1984. 73, 923-932). Briefly, 20 µl of a 0.4% (wt/vol) solution of Evans blue (Sigma) in sterile physiological saline was injected into a tail vein. Blood samples (10 µl) were collected at 10 min and 30 min after injection to measure disappearance kinetics. Tubes were centrifuged and the hematocrit was recorded. Evans blue concentration in the plasma was measured in duplicate as optical density using the 2-wavelength method.

### Total cell membrane isolation

Mouse kidney cortex was homogenized in a homogenizing buffer [20 mM Tris (pH 7.4/HCl), 5 mM MgCl₂, 5 mM NaH₂PO₄, 1 mM ethylenediaminetetraacetic acid (pH 8.0/NaOH), 80 mM sucrose, 1 mM phenyl-methylsulfonyl fluoride, 10 µg/ml leupeptin, and 10 µg/ml pepstatin] and subsequently centrifuged for 15 min at 4,000 g. Supernatants were transferred to a new tube and centrifuged for an additional 30 min at 16,000 g.

### Histology and morphologic evaluation of aorta and kidney

For histological analysis, kidney and thoracic aortas were fixed in 4% paraformaldehyde (PFA) overnight, processed for paraffin histology, sectioned at 5 µm thickness, and stained by routine methods, using hematoxylin/eosin (HE), von Kossa, Alizarin red, or periodic acid-Schiff (PAS) staining (Sigma-Aldrich). Quantification of von Kossa-stained aortas was performed using Image J software. The data were expressed as percentage of calcified regions relative to the total aortic area. Semi-quantitative pathohistologic scoring of glomerular lesions in the kidney was performed in a blind fashion. The total number of kidney glomeruli was counted, and the degree of glomerular damage was estimated at 400× magnification from the degree of mesangial expansion in PAS-stained sections as follows: <25%, 25-50%, 50-80%, >80% (MacKay K et al., Kidney Int., 1987. 32, 827-837).

### Laser capture microdissection (LCM)

Cryosections from femurs or kidney were quickly stained with HistoStain (Arcturus). Osteoblasts and osteocytes from cortical bone of the femur sections, and distal and proximal tubules from kidney sections were harvested using a Veritas (Arcturus) LCM system as described in Andrukhova O et al. (Bone, 2012. 51, 621-628), and stored at -80°C until used.

### RNA isolation and quantitative RT-PCR

Shock-frozen hearts were homogenized using TRI Reagent (Molecular Research Center). Total RNA was extracted with phenol/chloroform, precipitated using isopropanol, and then treated with RQ1 RNase-free DNase (Promega). RNA purity and quality was determined spectrophotometrically (BioPhotometer; Eppendorf). In samples harvested with LCM, RNA was extracted using the PicoPure RNA isolation kit (Qiagen), and RNA purity and quality was determined spectrophotometrically (BioPhotometer, Eppendorf) and via the 2100 Bioanalyzer (Agilent Technologies). After first-strand cDNA synthesis (iScript cDNA Synthesis Kit, BioRad), quantitative RT-PCR was performed on a Rotor-Gene 6000 (Corbett Life Science) using SsoFastTM EvaGreen PCR kit (Bio-Rad). A melting curve analysis was done for all assays to make sure that only a single PCR product was amplified. Primer sequences are available on request. Efficiencies were examined by standard curve. Gene expression data were corrected for efficiency and normalized to ornithine decarboxylase antizyme-1 (Oaz1) as house-keeping gene.

### Western blotting

Total kidney homogenates, kidney cortex homogenates or total cell membrane preparations were solubilized in Laemmli sample buffer, fractionated on SDS- PAGE (30 µg/well) and transferred to a nitrocellulose membrane (Thermo Scientific). Immunoblots were incubated overnight at 4°C with primary antibodies including polyclonal rabbit anti-TRPV5 (1:1,000, Alpha Diagnostics), polyclonal rabbit anti-NCC (1:3,000, Millipore), polyclonal sheep anti-phospho-NKCC1 Thr 203,207,212 (pNKCC1 T203, 207, 212; 1:1,000), polyclonal sheep anti-NKCC1 (1:1,000), antiphospho-NCC Thr 46, 50, 55 (pNCC T46, 50, 55; 1:1,000), polyclonal sheep anti-phospho-WNK4 Ser 47 (pWNK4 S47; 1:2,000), polyclonal sheep anti-phospho-WNK1 Ser 382 (pWNK1 S382; 1:2,000) (antibodies produced in house by Dario R. Alessi, University of Dundee, Dundee, UK), anti-α-ENaC (Novus Biologicals, 1:1,000), anti-β-ENaC and anti-γ-ENaC (Antikoerperonline.com, 1:1,500), and monoclonal mouse anti-β-actin (1:5,000, Sigma) in 2% (w/v) bovine serum albumin (BSA, Sigma) in a TBS-T buffer [150 mM NaCl, 10 mM Tris (pH 7.4/HCl), 0.2% (v/v) Tween-20]. After washing, membranes were incubated with horseradish peroxidase-conjugated secondary antibodies (Amersham Life Sciences). Specific signal was visualized by ECL kit (Amersham Life Sciences). The protein bands were quantified by Image Quant 5.0 software (Molecular Dynamics). Loading of the samples was normalized to Ponceau S stain. The expression levels were normalized to β-actin expression.

### Ex vivo experiments with kidney slices

The preparation of kidney slices for ex vivo experiments was performed as described in Andrukhova O et al. (EMBO J., 2014. 33, 229-246). Longitudinal 200-µm-thick live slices from freshly isolated kidneys of 3-month-old male WT and Klotho/VDR mice were prepared using a Leica VT1000 Vibratome (Leica Microsystems), and were used for ex vivo treatment with various substances as described below.

### Immunohistochemistry

For immunohistochemistry, 5-µm-thick paraffin sections of PFA-fixed kidneys or of human renal biopsies were prepared. Before immunohistochemical staining, dewaxed sections were pretreated with blocking solution containing 5% normal goat serum in PBS with 0.1% bovine serum albumin and 0.3% Triton X-100 for 60 min. Without rinsing, mouse kidney sections were incubated with rabbit anti-TRPV6 (Millipore, 1:400), sheep anti-NKCC1, sheep anti-pNKCC1 T203, 207, 212, (1:1000, antibodies produced in house by Dario R. Alessi) antibodies at 4°C overnight. Sections of human biopsies were incubated with sheep anti-human pNKCC1 T212, 217, anti-human pNCC T46, 50, 55, and anti-human pWNK1 S382 (1:1,000, antibodies produced in house by Dario R. Alessi). After washing, sections were incubated for 1.5 h with biotinylated goat anti-rabbit or donkey anti-sheep or anti-goat secondary antibodies (Invitrogen, 1:400), followed by incubation with horseradish peroxidase-labeled streptavidin (Vector), and DAB staining. Controls were performed by omitting the primary antibody. The slides were analyzed on a Zeiss Axioskop 2 microscope. For quantification of pWNK1, pNKCC1, and pNCC expression in human renal biopsies, the average staining intensity was analyzed in about 20 fields per biopsy at x400, using Image J.

### Intracellular Na⁺ and Ca²⁺ imaging

Longitudinal 200-µm-thick live slices of freshly isolated kidneys were prepared using a Leica VT1000 Vibratome (Leica Microsystems). For Na⁺ imaging, the kidney slices of WT and Klotho/VDR mice were incubated for 60 min at 37°C with 2 µM of the sodium-sensitive dye SBFI (Molecular Probes), diluted in DMSO (Merck Millipore International) and 20% Pluronic (Merck Millipore International). For Ca²⁺ imaging, the kidney slices were incubated for 30 min at 37°C with 2 µM of the calcium-sensitive dye Fluo-4 (Molecular Probes), diluted in DMSO and 20% Pluronic. Thereafter, the slices were washed two times for 20 min each in 0.1M PBS, and were subsequently incubated in vitro with vehicle (PBS), rFGF23 R176/179Q [(100 or 200 ng/ml) kindly provided by Amgen Inc., Thousand Oaks, CA, USA], PD173074 (Sigma-Aldrich) (10 nM for inhibiting FGFR1 and 25 nM for inhibiting FGFR1 and 3), pan FGFR inhibitor [(120 nM) FIIN1 hydrochloride, Tocris Bioscience, Bristol, UK)], WNK signalling inhibitor Closantel (30 ng/mL, generous gift of Dario R. Alessi) alone or combined at 37°C, 5% CO2 / 95% air humidified atmosphere for 2 h. For the inhibition of NCC or NKCC1 activity, tissue slices were incubated with 10 µM of chlorothiazide (CTZ, Sigma) or 15 µM bumetanide (BF, Sigma) or vehicle (PBS + 1% ethanol) alone or in combination with rFGF23 R176/179Q (200 ng/ml). For inhibition of TRPV activity, tissue slices were incubated with 1 µM (for TRPV5) or 10 µM (for TRPV6) of ruthenium red (Sigma) alone or in combination with rFGF23 (200 ng/ml). For visualization of intracellular Na⁺ and Ca²⁺ content, SBFI or Fluo-4 were excited by a Ti:sapphire laser (Chameleon, Coherent Inc.) at 820 nm. Images (512 × 512 pixels) were acquired every 30 sec at a depth of 60 - 80 µm. Fluorescence images were analyzed using Image J software. The whole epithelial layer of the distal tubules was selected by manually drawing the region of interest (ROI) to quantify intracellular Na⁺ and Ca²⁺ levels. Fluorescence intensity was quantified in 4-9 ROIs per image, and the ratio between the fluorescence intensity and the ROI area was calculated for each tubule. This ratio was used for all subsequent calculations.

### Total cell membrane isolation

Mouse kidney cortex was homogenized in a homogenizing buffer [20 mM Tris (pH 7.4/HCl), 5 mM MgCl₂, 5 mM NaH₂PO₄, 1 mM ethylenediaminetetraacetic acid (pH 8.0/NaOH), 80 mM sucrose, 1 mM phenyl-methylsulfonyl fluoride, 10 µg/ml leupeptin, and 10 µg/ml pepstatin] and subsequently centrifuged for 15 min at 4,000 g. Supernatants were transferred to a new tube and centrifuged for an additional 30 min at 16,000 g. For Western blotting analysis, samples were dissolved in loading buffer and stored at -80°C until analyzed.

### Statistical analyses

Statistics were computed using SPSS for Windows 17.0. The data were analyzed by 1-way analysis of variance (ANOVA) followed by Student-Newman-Keuls multiple comparison test. P values of less than 0.05 were considered significant. The data are presented as the mean ± SEM. For correlation analysis, bivariate Spearman rank correlation coefficients were calculated. A stepwise model of multiple regression analysis was performed to find explanatory models for the changes in heart-to-body weight ratio after CKD.

### Example 1: Induction of CKD in C57BL/6 mice.

In order to test susceptibility of C57BL/6 mice to 5/6 nephrectomy (5/6-Nx)- and angiotensin II (AngII) infusion-induced CKD, 5/6-Nx was performed and osmotic minipumps continuously releasing vehicle or AngII were thereafter implanted into male wild-type (WT) C57Bl/6 mice, which were then monitored over 8 weeks after 5/6-Nx. All mice were maintained on rescue diet.

Results: As shown in Figure 1 no significant differences in GFR, proteinuria, azotemia, or mean arterial pressure measured by arterial catheterization between AngII- and vehicle-treated 5/6-Nx mice at 4 and 8 weeks postsurgery were found.

These data indicate that 5/6-Nx is sufficient to induce an about 60% reduction in GFR in C57BL/6 mice on rescue diet, 8 weeks post-5/6-Nx.

### Example 2: Assessment of FGF23 and Klotho function during 5/6-Nx-induced CKD in mice.

Loss of Fgf23 or Klotho function leads to unleashed production of 1,25(OH)₂D₃ due to the lacking suppressive effect of Fgf23 on renal 1α-hydroxylase. Chronically elevated circulating 1,25(OH)₂D₃ levels result in hyperphosphatemia, hypercalcemia, soft tissue calcifications, and early mortality in Fgf23^{-/-} and Klotho^{-/-} mice. Consequently, the severe phenotype in Fgf23^{-/-} and Klotho^{-/-} mice can be completely rescued by genetically disrupting vitamin D receptor (VDR) signalling. Fgf23 and Klotho deficient mice were therefore crossed with VDR^{Δ/Δ} mice lacking a functioning VDR to generate Fgf23/VDR and Klotho/VDR compound mutants. When kept on a so-called "rescue diet" enriched with calcium, phosphate, and lactose, which serves to normalize Ca²⁺ and phosphorus homeostasis in VDR mutant mice, Fgf23/VDR and Klotho/VDR compound mutants are healthy, and can be analysed until old ages.

In order to assess whether *Fgf23* and *Klotho* deficiency influences disease progression after 5/6-Nx, CKD was induced by by 5/6-Nx in 3-month-old male WT, VDR, Fgf23/VDR, and Klotho/VDR mice.

Results: Figure 2 shows that mortality after CKD induction was low in WT, VDR and Fgf23/VDR mice, but substantial in Klotho/VDR compound mutants. In WT and VDR mutant mice, 5/6-Nx reduced GFR by ∼60% vs. Sham controls, 8 weeks postsurgery (Fig. 2B). Importantly, genetic ablation of Fgf23 in Fgf23/VDR compound mutants almost completely protected against the CKD-induced decline in renal and cardiac function, heart hypertrophy as measured by heart-to-body weight (HW/BW) ratio, volume expansion as measured by blood volume and end-diastolic pressure, hypertension, left ventricular functional impairment, aortic calcification, and glomerular lesions observed in WT and VDR CKD mice (Fig. 2B-D and Fig. 3A-C). In contrast to Fgf23/VDR CKD mice, Klotho/VDR CKD mice were not protected against disease progression, and actually showed the most severe glomerular lesions of all genotypes (Fig. 2B-D and Fig. 3A-C). A correlation analysis revealed that serum Fgf23, aldosterone, PTH, blood pressure, as well as serum Ca²⁺ and Na⁺ were correlated with HW/BW ratio (Table 1). However, blood volume showed the strongest association with HW/BW ratio (Fig. 2C), and remained the only significant predictor of HW/BW ratio in a stepwise model of multiple regression analysis (Table 2).

**Table 1: Correlation coefficient matrix for heart-to-body weight ratio, blood volume and selected serum parameters in Sham and CKD mice (Spearman rank correlation coefficients rS).**

| | **HW/BW** | **Fgf23** | **Volume** | **ALD** | **PTH** | **MAP** | **Se Na** | **Se Ca** | **Se Pi** |
|---|---|---|---|---|---|---|---|---|---|
| **HW/BW** | | 0.565** | 0.733** | 0.527** | 0.589** | 0.665** | 0.420* | 0.600** | 0.221 |
| **Fgf23** | 0.565** | | 0.548** | 0.883** | 0.812** | 0.634** | 0.019 | 0.445* | -0.117 |
| **Volume** | 0.733** | 0.548** | | 0.612** | 0.569** | 0.721** | 0.254 | 0.642** | 0.133 |
| ALD | 0.527** | 0.883** | 0.612** | | 0.826** | 0.682** | -0.041 | 0.461* | 0.057 |
| **PTH** | 0.589** | 0.812** | 0.569** | 0.826** | | 0.704** | 0.191 | 0.452* | 0.107 |
| **MAP** | 0.665** | 0.634** | 0.721** | 0.682** | 0.704** | | 0.295 | 0.629** | -0.032 |
| **Se Na** | 0.420* | 0.019 | 0.254 | -41 | 0.191 | 0.295 | | 0.294 | 0.113 |
| **Se Ca** | 0.600** | 0.445* | 0.642** | 0.461* | 0.452* | 0.629** | 0.294 | | 0.310 |
| **Se Pi** | 0.221 | -0.117 | 0.133 | 0.057 | 0.107 | -0.032 | 0.113 | 0.310 | |

Heart weight to body weight - HW/BW; serum Fgf23 - Fgf23; blood volume - volume; serum aldosterone - ALD; serum parathyroid hormone - PTH; serum - Se. Mean arterial pressure - MAP. * p < 0.05, ** p < 0.01.

**Table 2: Stepwise multiple regression analysis of the relationship between heart-to-body weight ratio, blood volume and selected serum parameters in Sham and CKD animals.**

| ***Dependent variable*** | ***Independent variable*** | ***Partial regression coefficient*** | ***β-coefficient*** | ***P level*** | ***R² for model*** | ***P level*** |
|---|---|---|---|---|---|---|
| **HW/BW** | Volume | 1.140 | 0.378 | 0.012 | 0.719 | 0.003 |

Heart weight to body weight - HW/BW; serum Fgf23, blood volume (volume), serum aldosterone, serum PTH, serum calcium, serum phosphate, and mean arterial pressure were used as independent variables in the model.

The data therefore suggests that CKD-induced cardiac hypertrophy is mainly driven by increased volume overload.

### Example 3: Assessment of the accelerated CKD progression in Klotho/VDR compound mutants.

In order to assess differences in mortality, disease progression and vascular calcification between Fgf23/VDR and Klotho/VDR CKD mice, mineral homeostasis was examined.

Results: Figure 4 shows hyperphosphatemia, hypocalcemia, renal Ca²⁺ and Na⁺ wasting, increased PTH and aldosterone levels, together with reduced renal expression of TRPV5 and NCC and augmented expression of αENaC in Sham Fgf23/VDR and Klotho/VDR compound mutants relative to WT and VDR controls. In WT and VDR mutant mice, 5/6-Nx induced hypercalcemia, hypernatremia, reduced renal excretion of phosphate and Ca²⁺, as well as increased serum levels of PTH, aldosterone, and Fgf23 (Fig. 4A-C). Notably, 5/6-Nx resulted in hyperphosphatemia in WT but not in VDR mutant mice (Fig. 4A-C). Since disease progression was not different between VDR mutant and WT CKD mice (Fig. 2B-D), this unexpected but interesting finding suggests that hyperphosphatemia is not a key driving factor for disease progression after 5/6-Nx. In line with the higher serum levels of Fgf23, PTH, and aldosterone, renal expression of fully glycosylated TRPV5, NCC, and αENaC was increased in WT and VDR CKD mice (Fig. 4D). Significant differences between Fgf23/VDR and Klotho/VDR CKD mice were found only for total serum Ca²⁺, urinary Ca²⁺ excretion, and serum aldoste-rone concentration. Interestingly, 5/6-Nx failed to induce renal Ca²⁺ retention in Fgf23/VDR in contrast to Klotho/VDR mice (Fig. 4A-C). However, TRPV5 expression in the kidney was upregulated in a similar fashion in Fgf23/VDR and Klotho/VDR CKD mice (Fig. 4D).

While renal Klotho protein expression remained unchanged in CKD mice of all genotypes (Fig. 3D), Klotho/VDR compound mutants showed the highest circulating intact Fgf23 levels among all CKD groups (Fig. 4C). Similarly, Fgf23 mRNA expression as evidenced by laser capture microdissection, and Fgf23 protein expression assessed by Western blotting showed the strongest upregulation in bones of Klotho/VDR CKD animals compared to the other CKD groups (Fig. 3E-F). Fgf23 expression was augmented in both osteoblasts and osteocytes in Klotho/VDR CKD animals (Fig. 3E-F).

### Example 4: Pharmacological inhibition of Fgf23 largely protects against the CKD-induced renal and cardiovascular pathology.

To answer the question whether the increased circulating Fgf23 was the cause of accelerated CKD progression in Klotho/VDR compound mutants, WT, VDR and Klotho/VDR CKD mice were treated with low doses of blocking anti-FGF23 antibody (anti-FGF23Ab, 50 µg per mouse, two times per week) or control antibody (Co Ab) over 8 weeks post-surgery. High-dose anti-FGF23 therapy was shown to be actually detrimental in a rat CKD model, increasing mortality and aortic calcification by inducing hyperphosphatemia together with excessive stimulation of vitamin D hormone production (Hoorn, E.J. & Ellison, D.H., Exp. Cell Res., 2012, 318, 1020-1026). The rationale behind this experiment was that pharmacological neutralization of Fgf23 would ameliorate CKD progression only in case that elevated circulating Fgf23 was driving disease progression, but not if lack of Klotho was the cause of aggravated disease progression in Klotho/VDR CKD mice.

Results: Figure 5 shows that treatment of CKD mice with low dose anti-FGF23Ab reduced circulating Fgf23 in CKD mice to Sham control levels in all genotypes, but did not completely eliminate circulating Fgf23. Notably, anti-Fgf23 treatment reduced the increased mortality in Klotho/VDR CKD mice to WT and VDR control levels (Fig. 5B), suggesting that increased circulating Fgf23 is indeed the cause of increased mortality in Klotho/VDR CKD mice. In analogy to the genetic loss-of-function model, treatment of WT, VDR and Klotho/VDR CKD mice with anti-FGF23Ab partially or completely protected against the CKD-induced weight loss, reduction in GFR, albuminuria, hypercalcemia, aortic calcification, hypertension, heart hypertrophy, and left ventricular functional impairment (Fig. 5C-E, Fig. 6). Importantly, anti-Fgf23 treatment reduced end-diastolic pressure in CKD mice of all genotypes to Sham control levels (Fig. 5E), corroborating the notion that Fgf23-induced volume overload is a major pathogenetic factor in CKD progression. Since anti-Fgf23 therapy reduced renal tubular Ca²⁺ and Na⁺ reabsorption also in Klotho/VDR CKD mice (Fig. 5F), it seems that Fgf23 must have Klotho-independent effects on Ca²⁺ and Na⁺ homeostasis. Although anti-Fgf23 therapy might theoretically aggravate hyperphosphatemia in CKD mice, we did not observe anti-Fgf23 therapy-induced changes in serum phosphate levels or urinary phosphate excretion in any of the genotypes, despite the fact that anti-Fgf23 therapy lowered circulating intact PTH in CKD mice (Fig. 6).

The data suggests that in order to exploit the strikingly beneficial effects of lowering blood Fgf23 concentrations in CKD, it would be necessary to carefully titrate the dose of anti-FGF23 Ab to prevent the toxic effects of Fgf23 over-suppression.

**Example 5:** Protein expression analysis of the main Ca²⁺ and Na⁺ channels in the distal nephron as well as expression of activated WNK1 and WNK4, two of the most important regulators of intracellular ion channel trafficking in distal renal tubules (Nilius et al., Br. J. Pharmacol., 2001. 134, 453-462).

In order to assess how excessive intact Fgf23 can modulate renal Ca²⁺ and Na⁺ handling in CKD mice in a Klotho-independent manner, protein expression of αENaC, γENaC, TRPV5, NCC, phospho (p)-NCC, pNKCC1 (Na+-potassium-chloride cotransporter-1), pWNK1, and pWNK4 was performed.

Results: Figures 7 and 8 show that 5/6-Nx upregulated renal protein expression of αENaC, γENaC, TRPV5, NCC, phospho (p)-NCC, pNKCC1 (Na⁺-potassium-chloride cotransporter-1), pWNK1, and pWNK4 in all genotypes. Interestingly, TRPV6 expression was profoundly upregulated in Klotho/VDR, but not in WT or VDR CKD mice (Fig. 7 and 8). Anti-Fgf23 Ab therapy reduced αENaC, NCC, pNCC, pNKCC1, pWNK1, and pWNK4 in all genotypes (Fig. 7 and 8). In addition, anti-Fgf23 therapy reduced the augmented TRPV6 expression in Klotho/VDR CKD mice to Sham control levels (Fig. 7). In contrast, γENaC and TRPV5 expression remained unchanged in anti-Fgf23-treated CKD mice (Fig. 7).

Collectively, these results indicate that excessive circulating Fgf23 activates WNK signalling, and upregulates the expression and activation of Ca²⁺- and Na⁺-transporting molecules in a partially Klotho-independent manner in CKD mice.

### Example 6: Serum intact FGF23 levels are associated with WNK activation in the kidney of human CKD patients.

In order to assess whether increased circulating intact FGF23 is also associated with WNK activation in humans, a prospective study in ten patients with CKD stages 2 - 5 undergoing diagnostic renal biopsies was performed.

Results: pNCC, pNKCC1, and pWNK1 expression by immunohistochemistry was quantified in renal biopsies. pNCC expression was not significantly correlated with serum intact FGF23 (rₛ=0.297; P=0.407). However, Figure 9 shows a strong association between serum intact FGF23 and distal renal tubular pWNK1 and pNKCC1 expression.

This data suggests that FGF23-induced WNK activation in distal renal tubules is a pivotal mechanism in human CKD pathophysiology.

### Example 7: Very high concentrations of FGF23 activate WNK signalling in a Klotho-independent manner in kidney slices ex vivo.

In order to examine the Klotho-independent effects of excessive Fgf23 signalling on TRPV6 expression and phosphorylation of NCC, NKCC1 and WNK4, and to rule out indirect effects of altered circulating aldosterone and PTH levels in CKD mice, 200-µm-thick kidney slices isolated from WT and Klotho/VDR animals were treated with two different concentrations of recombinant FGF23 (rFGF23), 100 ng/ml (high) and 200 ng/ml (very high), for 2 h ex vivo, and analysed protein expression of the target molecules by Western blotting.

Results: One-hundred ng/ml rFGF23 increased pWNK1, pWNK4, and TRPV5 expression in WT, but with the exception of pWNK1 not in Klotho/VDR kidney slices (Fig. 10A-C). In contrast, Figure 10 further shows that 200 ng/ml of rFGF23 upregulated pWNK1, pWNK4, pNKCC1, and TRPV6 expression in both WT and Klotho/VDR kidney slices, indicating that very high concentrations of FGF23 are able to activate WNK signalling, NKCC1 phosphorylation, and TRPV6 expression in a Klotho-independent fashion in vitro.

### Example 8: Very high concentrations of FGF23 stimulate distal tubular calcium and sodium transport in kidney slices by Klotho- and FGFR1-independent activation of NKCC1 and TRPV6 channels.

To gain more insight into the functional role of the rFGF23-induced increase in distal renal tubular ion channel expression and the FGF receptors involved, we performed intracellular Ca²⁺ and Na⁺ imaging by 2-photon microscopy in Fluo-4- or SFBI-loaded and subsequently rFGF23-treated (100 or 200 ng/ml for 2 h), 200-µm-thick renal slices prepared from WT and Klotho/VDR mice.

Results: Figure 11A-D shows that rFGF23 increased intracellular Ca²⁺ and Na⁺ fluorescence in WT distal tubules at both concentrations, whereas only 200 ng/ml rFGF23 was able to increase intracellular Ca²⁺ and Na⁺ fluorescence in kidney slices of Klotho/VDR mice. The FGF23-induced Ca²⁺ uptake was reversed in WT kidney slices by co-treatment with the TRPV blocker ruthenium red at 1 µM. Ruthenium red at 1 µM is known to block only TRPV5, but not TRPV6 channel function (Nilius B et al., Br. J. Pharmacol., 2001. 134, 453-462). In contrast, co-treatment with 10 µM ruthenium red was necessary to antagonize the rFGF23-induced increase in distal tubular Ca²⁺ uptake in Klotho/VDR kidney slices (Fig. 11A and C), suggesting the involvement of TRPV6 in this process. The inhibitory effects of the specific NCC and NKCC1 blockers chlorothiazide (CTZ) or bumetanide (BF) respectively (Monroy A et al., Am. J. Physiol Renal Physiol, 2000. 279, F161-F169; Isenring P et al., J. Biol. Chem., 1998. 273, 11295-11301), was tested to analyse the molecules involved in the FGF23-induced Na⁺ uptake. Figure 11B and D show that both, CTZ and BF, decreased SFBI fluorescence intensity in distal tubules of rFGF23-treated WT kidney slices by approximately 50%, whereas the Na⁺ influx in Klotho/VDR kidney slices was inhibited exclusively by the NKCC1 inhibitor BF. To identify the FGFRs involved in the rFGF23-induced increase Ca²⁺ and Na⁺ uptake, kidney slices of WT and Klotho/VDR mice were co-treated with 200 ng/ml rFGF23 and an FGFR1 inhibitor (iFGFR1) or a pan FGFR inhibitor (iFGFR pan). FGFR1 inhibition had no effect on the rFGF23-induced increase in Ca²⁺ and Na⁺ fluorescence in Klotho/VDR kidney slices, but largely blocked the effect of rFGF23 in WT kidney slices. In contrast, Figure 11A-D shows that the iFGFR pan inhibitor completely blocked the rFGF23-induced increase in intracellular Ca²⁺ and Na⁺ fluorescence in both WT and Klotho/VDR kidney slices.

These data show that excessive concentrations of rFGF23 enhance distal tubular Ca²⁺ and Na⁺ uptake in vitro by a combination of Klotho-dependent FGFR1-mediated activation of TRPV5 and NCC, and of Klotho- and FGFR1-independent activation of TRPV6, NCC and NKCC1, respectively.

### Example 9: Assessment of renal FGFR mRNA expression and effects of FGFR1/3 and/or WNK signalling inhibition on distal tubular FGF23-mediated calcium and sodium uptake in kidney slices of Klotho/VDR mutants.

In this example it was assessed whether the Klotho-independent FGF23 signalling pathway is mediated through FGFR3 and/or 4, given that FGFR2 expression was low in distal renal tubules (Figure 12A). To dissect between the latter receptors, Klotho/VDR kidney slices were treated with rFGF23 alone or in combination with an FGFR1/FGFR3 inhibitor and the WNK signalling inhibitor closantel.

Results: Figure 12B-C shows that closantel abrogated the FGF23-induced upregulation in distal tubular Ca²⁺ and Na⁺ uptake, demonstrating the essential role of WNK activation in this process. FGFR1/3 inhibition partially blocked the FGF23-induced Ca²⁺ influx, but completely blocked the FGF23-induced increase in Na⁺ uptake (Fig. 12B-C).

These data suggest that the Klotho-independent activation of intracellular Ca²⁺ uptake in distal renal tubules by excessive FGF23 in vitro is mediated through FGFR3 and 4, whereas the Klotho-independent FGF23-induced stimulation of Na⁺ uptake is mainly mediated by FGFR3.

### Example 10: Assessment of the effects of oral closantel administration in CKD mice.

In order to assess whether WNK signalling inhibition is able to ameliorate CKD disease progression in vivo, WT Sham and 5/6-Nx mice on rescue diet were treated over 8 weeks with closantel orally via the diet. To maintain constant plasma levels, the mice received closantel-containing food once a week, using the dosing regimen as described in the materials and methods. The pharmacokinetic model underlying the calculation of the dosing regimen was based on the assumption of a plasma half-life of 14 days.

Results: As shown in Figure 13A-B, closantel largely prevented the CKD-driven decline in GFR, as well as the CKD-induced increase in HW/BW ratio, blood volume, and blood pressure by downregulation of renal pNCC, pNKCC, and pWNK1 expression, which resulted in increased urinary excretion of Na⁺ in CKD mice.

These data indicate that the small molecule WNK signalling inhibitor closantel may present a new therapeutic strategy for CKD patients.

## Claims

1. A WNK signalling inhibitor for use in the prevention and/or treatment of disorders characterized and/or caused by high levels of serum fibroblast growth factor 23 (FGF23), especially chronic kidney disease (CKD).

2. A WNK signalling inhibitor for use according to claim 1, wherein the WNK signalling inhibitor is selected from the group consisting of Closantel (N-[5-chloro-4-[(4-chlorophenyl)-cyanomethyl]-2-methylphenyl]-2-hydroxy-3,5-diiodobenzamide), STOCK1S-14279 (N-[4-(1-bromonaphthalen-2-yl)oxy-3-chlorophenyl]-3,5-dichloro-2-hydroxybenzamide), STOCK1S-50699 ((2Z)-3-ethyl-2-[[(3Z)-3-[(E)-3-(3-ethyl-1,3-benzothiazol-3-ium-2-yl)prop-2-enylidene]-5-methylcyclohexen-1-yl]methylidene]-1,3-benzothiazole), STOCK2S-26016 (7-Ethoxy-N3-(2-furanylmethyl)-3,9-acridinediamine), WNK463 (N-tert-butyl-3-[1-[5-[5-(trifluoromethyl)-1,3,4-oxadiazol-2-yl]pyridin-2-yl]piperidin-4-yl]imidazole-4-carboxamide), or any combination thereof, preferably Closantel or a pharmaceutically acceptable salt thereof.

3. A WNK signalling inhibitor for use according to claim 1 or 2, wherein said disorder is selected from the group consisting of tumor-induced osteomalacia (TIO), X-linked hypophosphatemia, autosomal dominant hypophosphatemic rickets (ADHR), McCune-Albright syndrome, linear nevus sebaceous syndrome, acute myocardial infarction, and CKD, preferably CKD.

4. A WNK signalling inhibitor for use according to any one of claims 1 to 3, wherein said inhibitor is for use in humans and/or non-human animals, particularly domesticated companion animals, e.g. dogs and cats.

5. Pharmaceutical composition for use in the prevention and treatment of disorders characterized and/or caused by high levels of serum FGF23, especially CKD, comprising one or more WNK signalling inhibitor as defined in any one of claims 1 to 4.

6. Pharmaceutical composition for use according to claim 5, wherein the composition further comprises a pharmaceutically acceptable excipient and/or solvent.

7. Pharmaceutical composition according to claim 5 or 6, wherein the pharmaceutically acceptable excipient may be selected from the group consisting of surfactants, preferably oleic acid, thickening agents, salts, buffers, or any other inert excipient commonly used in pharmaceutical industry.

8. Pharmaceutical composition according to any one of claims 5 to 7, wherein the pharmaceutically acceptable solvent is selected from the group consisting of ethanol, benzyl alcohol, isopropanol, butanol, or a mixture thereof, propylene glycol, diethylene glycol monoethyl ether (transcutol), ethylene glycol, butylene glycol, polyvinylpyrrolidone, polyoxypropylene/polyoxyethylene, polyethylene glycol, or the like, preferably propylene glycol, diethylene glycol monoethyl ether or a mixture thereof.

9. Pharmaceutical composition according to any one of claims 5 to 8, wherein said composition may be in form of tablets, lozenges, hard or soft capsules, aqueous or oily suspensions, emulsions, dispersible powders or granules, syrups or elixirs, or sterile aqueous or oily solutions.

10. Pharmaceutical composition according to any one of claims 5 to 9, wherein the pharmaceutical composition is suitable for oral and/or parenteral administration.

11. Pharmaceutical composition according to any one of claims 5 to 10 for use in humans and/or non-human animals, particularly domesticated companion animals.

12. Pharmaceutical composition according to any one of claims 5 to 11 for use in a method for preventing and/or treating disorders **characterized by** elevated serum FGF23 levels, especially CKD, comprising administering an effective amount of said composition to a subject in need thereof, wherein the subject may be human or a non-human animal.
